# EUROPEAN PATENT APPLICATION

(11) **EP 4 840 949 A1**
(43) Date of publication of application: **18.11.2026**
(21) Application number: 25745365.4
(22) Date of filing: 23.01.2025
(51) Int. Cl.: C12N 9/10, C12N 15/77, C12N 15/70, C12P 19/56

(54) **NOVEL MUTANT GLYCOSYLTRANSFERASE POLYPEPTIDE AND METHOD FOR PRODUCING STEVIOL GLYCOSIDES USING SAME**

(30) Priority: 24.01.2024 KR 20240011204
(71) Applicant: CJ Cheiljedang Corporation, Seoul 04560 (KR)
(72) Inventor: LEE, Misun, Seoul 04560 (KR); KIM, Jungeun, Seoul 04560 (KR); JEONG, Goo-Yeong, Seoul 04560 (KR); PARK, Sunghee, Seoul 04560 (KR)
(74) Representative: Meissner Bolte Partnerschaft mbB
(86) International application number: PCT/KR2025/001347
(87) International publication number: WO 2025/159542

(57) **Abstract**

Provided are a novel variant polypeptide; a polynucleotide encoding the variant polypeptide; a vector comprising the polynucleotide; a microorganism comprising any one or more of the variant polypeptide, the polynucleotide encoding the variant polypeptide, and the vector comprising the polynucleotide; a method of producing a steviol glycoside using the variant polypeptide or the microorganism; a composition for producing any one or more of rebaudioside D and rebaudioside M using the same; and use of the microorganism in producing any one or more of rebaudioside D and rebaudioside M.

## Description

### [Technical Field]

The present disclosure relates to a novel variant polypeptide; a polynucleotide encoding the variant polypeptide; a vector comprising the polynucleotide; a microorganism comprising any one or more of the variant polypeptide, the polynucleotide encoding the variant polypeptide, and the vector comprising the polynucleotide; a method of producing a steviol glycoside, the method comprising the step of transferring glucose to rebaudioside A by reacting rebaudioside A with the nucleotide diphosphate-linked glucose in the presence of the variant polypeptide or the microorganism; a composition for producing any one or more of rebaudioside D and rebaudioside M using the same; and use of the microorganism in producing any one or more of rebaudioside D and rebaudioside M.

### [Background Art]

As the World Health Organization (WHO) recommends lowering the amount of daily sugar intake due to concerns about diseases (obesity) caused by sugar consumption, various policies aimed at reducing the amount of sugar intake are actively being discussed under the leadership of governments, mainly in developed countries. Therefore, the demand for various alternative sweeteners to replace sugar and high fructose is increasing in the market, and alternative sweeteners are continuously being developed and commercialized.

From this perspective, customers' need for natural sweeteners has been steadily increasing due to concerns about the safety of synthetic sweeteners. However, due to limitations in the taste properties of natural sweeteners, such as their unique off-flavor and off-smell, they cannot fully replace the existing low-calorie and zero-calorie products based on synthetic sweeteners.

In this regard, a natural high-intensity sweetener that has received considerable attention in recent years is the stevia sweetener extracted from the leaves of Stevia (US Patent Publication No. 2023-0270146). Among the sweetening components of stevia or extracts thereof, rebaudioside D and M have low bitterness and excellent sweetness, and thus they are valuable as alternative sweeteners. However, they exist only in very small amounts in stevia leaves, and thus there is a disadvantage in that the method of extracting and purifying the same from the leaves requires high costs.

Accordingly, there is a growing need for research on new enzymes required for the mass-production of rebaudioside D and M and their preparation using the same.

### [Disclosure]

### [Technical Problem]

A problem to be solved in the present disclosure is to provide a variant polypeptide having glycosyltransferase activity; a polynucleotide encoding the variant polypeptide; a vector comprising the polynucleotide; a microorganism comprising any one or more of the polypeptide, the polynucleotide, and the vector; a method of producing any one or more of rebaudioside D and rebaudioside M, the method comprising the step of culturing the microorganism in a medium; a method of producing a steviol glycoside, the method comprising the step of transferring glucose to rebaudioside A by reacting rebaudioside A with the nucleotide diphosphate-linked glucose in the presence of the variant polypeptide and/or the microorganism; a composition for producing any one or more of rebaudioside D and rebaudioside M using the same; and use of the microorganism in producing any one or more of rebaudioside D and rebaudioside M.

### [Technical Solution]

An object of the present disclosure is to provide a variant polypeptide having glycosyltransferase activity, the variant polypeptide comprising any one or more of the following substitutions: a substitution of an amino acid corresponding to position 92 of the amino acid sequence of SEQ ID NO: 1 with tryptophan; a substitution of an amino acid corresponding to position 208 with alanine or methionine; a substitution of an amino acid corresponding to position 380 with histidine, threonine, alanine, or glycine; and a substitution of an amino acid corresponding to position 381 with phenylalanine, serine, alanine, or asparagine.

Another object of the present disclosure is to provide a polynucleotide encoding the variant polypeptide of the present disclosure.

Still another object of the present disclosure is to provide a vector comprising the polynucleotide.

Still another object of the present disclosure is to provide a microorganism comprising any one or more of the variant polypeptide of the present disclosure, the polynucleotide encoding the variant polypeptide, and the vector comprising the polynucleotide.

Still another object of the present disclosure is to provide a method of producing a steviol glycoside, the method comprising the step of transferring glucose to rebaudioside A by reacting rebaudioside A with the nucleotide diphosphate-linked glucose in the presence of the variant polypeptide and/or the microorganism, wherein the steviol glycoside is any one or more of rebaudioside D and rebaudioside M.

Still another object of the present disclosure is to provide a composition for producing any one or more of rebaudioside D and rebaudioside M, the composition comprising any one or more of the following: the variant polypeptide of the present disclosure, the polynucleotide encoding the variant polypeptide; the microorganism comprising any one or more of the variant polypeptide, the polynucleotide encoding the variant polypeptide, and the vector comprising the polynucleotide; and a culture of the microorganism.

Still another object of the present disclosure is to provide use of the microorganism in producing rebaudioside D and rebaudioside M.

Still another object of the present disclosure is to provide use of the composition in producing rebaudioside D and rebaudioside M.

### [Advantageous Effects]

When a variant polypeptide of the present disclosure or a microorganism comprising the same is used, it is possible to produce rebaudioside D and/or M in high yields, compared to an unmodified polypeptide or microorganism.

### [Brief Description of the Drawing]

FIG. 1 shows the results of comparing RebD synthesis ability using 10 types of single variants of improved enzymes of the present disclosure;
FIG. 2 shows the results of comparing RebD synthesis ability using 16 types of dual variants of improved enzymes of the present disclosure; and
FIG. 3 shows the results of comparing RebD synthesis ability using 10 types of complex variants (double mutations or more) of improved enzymes of the present disclosure.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

An aspect of the present disclosure provides a variant polypeptide having glycosyltransferase activity, wherein any one or more of the amino acids corresponding to positions 92, 208, 380, and 381 of an amino acid sequence of SEQ ID NO: 1 are substituted with different amino acids.

In one embodiment, the amino acid sequence of SEQ ID NO: 1 of the present disclosure and/or the variant polypeptide may be glycosyltransferase, specifically, UDP-glycosyltransferase (uridine diphosphate glycosyltransferase; UGT), and more specifically, UDP-glycosyltransferase B (UGT-B), but is not limited thereto.

As used herein, the term "glycosyltransferase" refers to an enzyme having an activity that transfers a monosaccharide moiety from a glycosyl donor to a glycosyl acceptor molecule, and UDP-glycosyltransferase refers to an enzyme that utilizes a nucleotide diphosphate-linked glucose (e.g., UDP-glucose) as a glycosyl donor. In the present disclosure, glycosyltransferase may be used interchangeably with "UDP-glycosyltransferase" and "UGT".

As used herein, the term "UDP-glycosyltransferase B" may refer to an enzyme capable of converting rebaudioside A (RebA) into rebaudioside D (RebD) using a nucleotide diphosphate-linked glucose as a glycosyl donor. Hereinbelow, in the present disclosure, UDP-glycosyltransferase B may be referred to interchangeably as glycosyltransferase B and UGT-B.

As used herein, the term "UDP-glycosyltransferase A" may refer to an enzyme capable of converting stevioside into rebaudioside A and/or rebaudioside D into rebaudioside M (RebM) using a nucleotide diphosphate-linked glucose as a glycosyl donor. Hereinbelow, in the present disclosure, UDP-glycosyltransferase A may be referred to interchangeably as glycosyltransferase A and UGT-A.

With regard to the variant polypeptide of the present disclosure, any one or more of the amino acids corresponding to positions 92, 208, 380, and 381 of the amino acid sequence of SEQ ID NO: 1, as a parent sequence, may be substituted with different amino acids.

As used herein, the parent sequence refers to a reference sequence into which a modification for preparing the variant polypeptide is introduced. In other words, the parent sequence may be a target into which substitution mutations of the present disclosure are introduced. The parent sequence may be naturally occurring or wild-type, or a variant in which one or more substitutions, insertions, or deletions have occurred in the natural or wild-type sequence, or may be an artificially synthesized sequence.

A polypeptide comprising the parent sequence of the present disclosure may comprise the amino acid sequence of SEQ ID NO: 1 or an amino acid sequence having 70% or more homology or identity thereto. In addition, even though a part of the amino acid sequence of SEQ ID NO: 1 is deleted or substituted, or another sequence is added to the amino acid sequence of SEQ ID NO: 1, as long as it is a protein exhibiting a function corresponding to glycosyltransferase activity, it may be comprised in the polypeptide comprising the parent sequence. Specifically, the polypeptide comprising the parent sequence may have or comprise the amino acid sequence of SEQ ID NO: 1 or an amino acid sequence having at least 70%, 80%, 85%, 90%, 95%, 96%, 97%, 98%, or 99% or more homology or identity thereto, or may consist of the amino acid sequence, or may essentially consist of the amino acid sequence. For example, the parent sequence of the present disclosure may be a polypeptide having or comprising the amino acid sequence of SEQ ID NO: 1, or consisting of the amino acid sequence or essentially consisting of the amino acid sequence, but is not limited thereto.

For example, SEQ ID NO: 1 of the present disclosure may be SEQ ID NO: 1 of US Patent No. 11,414,690, and US Patent No. 11,414,690 is incorporated herein by reference.

Further, a sequence of a polynucleotide encoding a polypeptide comprising a parent sequence having the amino acid sequence of SEQ ID NO: 1 or an amino acid sequence with 70% or more homology or identity thereto may be obtained, for example, based on codon information known in the art. For example, the polypeptide comprising the parent sequence may be encoded by a polynucleotide having or comprising a nucleotide sequence having 70% or more, 75% or more, 80% or more, 85% or more, 90% or more, 95% or more, 96% or more, 97% or more, 98% or more, or 99% or more homology or identity to the sequence of SEQ ID NO: 2 and/or SEQ ID NO: 127, or consisting of or essentially consisting of the nucleotide sequence, but is not limited thereto.

In the present disclosure, the polynucleotide (or gene) comprising the nucleotide sequence(s) of SEQ ID NO: 2 and/or SEQ ID NO: 127 may be used interchangeably with the polynucleotide (or gene) having or consisting of the nucleotide sequence(s) of SEQ ID NO: 2 and/or SEQ ID NO: 127.

Meanwhile, in the present disclosure, the expression "an amino acid corresponding to a specific position of (in) a specific amino acid sequence is substituted with a different amino acid" may be interpreted to mean that the amino acid sequence is used as a reference sequence.

As used herein, the term "reference sequence" means a sequence that serves as a reference for specifying the position N in a specific amino acid sequence. For example, when a specific amino acid sequence and a reference sequence are aligned through a sequence alignment known in the art, and based on this, when each amino acid residue of the specific amino acid sequence is numbered with reference to the numerical position of the amino acid residue of the reference sequence, the position of the amino acid corresponding to the position N of the reference sequence may be determined within the specific amino acid sequence.

As used herein, the term "position N" in an amino acid sequence may comprise an amino acid position corresponding to the position N, in addition to the position N. Specifically, the position N may comprise an amino acid position corresponding to an arbitrary amino acid residue in a mature polypeptide, disclosed in a specific amino acid sequence (e.g., reference sequence).

As used herein, the term "corresponding to" means an amino acid residue at a position listed in a polypeptide, or an amino acid residue similar to, identical to, or homologous to an amino acid residue listed in a polypeptide. Identifying the amino acid at the corresponding position may be determining a specific amino acid in a sequence that refers to a specific sequence. As used herein, the "corresponding region" generally refers to a similar or corresponding position in an amino acid sequence of a related protein or a reference protein.

In the present disclosure, the amino acid sequence of SEQ ID NO: 1 may be used as a reference sequence in determining the position of an amino acid in an arbitrary amino acid sequence.

In other words, the amino acid sequence of SEQ ID NO: 1 disclosed herein may be used to determine the corresponding amino acid residues in an arbitrary polypeptide. Unless indicated otherwise in the present disclosure, residues of a particular amino acid sequence are numbered based on the amino acid sequence of SEQ ID NO: 1.

For example, an arbitrary amino acid sequence is aligned with the amino acid sequence of SEQ ID NO: 1, and based on the alignment, each amino acid residue of the arbitrary amino acid sequence may be numbered with reference to the numerical position of the amino acid residue corresponding to the amino acid residue of SEQ ID NO: 1. For example, a sequence alignment algorithm such as that described herein may identify the position of an amino acid or a position where modifications such as substitutions, insertions, deletions, etc. occur, compared to a query sequence (also referred to as a "reference sequence").

In such alignment, for example, the Needleman-Wunsch algorithm (Needleman and Wunsch, 1970, J. Mol. Biol. 48: 443-453), the Needle program of the EMBOSS package (EMBOSS: The European Molecular Biology Open Software Suite, Rice et al., 2000), Trends Genet. 16: 276-277), etc. may be used, but is not limited thereto, and a sequence alignment program, a pairwise sequence comparison algorithm, etc., which known in the art, may be appropriately used.

Further, the corresponding amino acid residue in another polypeptide may be identified by multiple sequence alignment. Examples of the multiple sequence alignment known in the art comprise programs such as MUSCLE (multiple sequence comparison by log-expectation; version 3.5 or later; Edgar, 2004, Nucleic Acids Research 32: 1792-1797), MAFFT (6.857 or later; Katoh and Kuma, 2002, Nucleic Acids Research 30: 3059-3066; Katoh et al., 2005, Nucleic Acids Research 33: 511-518; Katoh and Toh, 2007, Bioinformatics 23: 372-374; Katoh et al., 2009, Methods in Molecular Biology 537: 39-64; Katoh and Toh, 2010, Bioinformatics 26: 1899-1900), and EMBOSS EMMA employing ClustalW (1.83 or later; Thompson et al., 1994, Nucleic Acids Research 22 : 4673-4680), etc., and their respective default parameters may be used, but are not limited thereto.

In addition, when polypeptides diverged from the mature polypeptide of SEQ ID NO: 1 fail to detect their relationship by traditional sequence-based comparison, other pairwise sequence comparison algorithms may be used (Lindahl and Elofsson, 2000, J. Mol. Biol. 295: 613-615). Higher sensitivity in sequence-based searching may be attained using search programs that utilize probabilistic representations of polypeptide families (profiles) to search databases. For example, the PSI BLAST program generates profiles through an iterative database search process and is capable of detecting remote homologs (Atschul et al., 1997, Nucleic Acids Res. 25: 3389-3402). Even greater sensitivity may be achieved if the family or superfamily for the polypeptide has one or more representatives in the protein structure databases. Programs such as GenTHREADER (Jones, 1999, J. Mol. Biol. 287: 797-815; McGuffin and Jones, 2003, Bioinformatics 19: 874-881) utilize information from a variety of sources, such as PSI BLAST, secondary structure prediction, structural alignment profiles, and solvation potentials, as input to a neural network that predicts the structural fold for a query sequence. Similarly, the method of Gough et al., 2000, J. Mol. Biol. 313: 903-919 may be used to align a sequence of unknown structure with the superfamily models present in the SCOP database. These alignments may be used in sequence to generate homology models for the polypeptides, and such models may be assessed for accuracy using a variety of tools developed for that purpose.

For proteins of known structure, several tools and resources are available for retrieving and generating structural alignments. For example, the SCOP superfamilies of proteins have been structurally aligned, and those alignments are accessible and downloadable. Two or more protein structures may be aligned using a variety of algorithms such as the distance alignment matrix (Holm and Sander, 1998, Proteins 33: 88-96) or combinatorial extension (CE) (Shindyalov and Bourne, 1998, Protein Engineering 11: 739-747). Implementation of these algorithms may be additionally utilized to query structure databases with a structure of interest in order to discover possible structural homologs (Holm and Park, 2000, Bioinformatics 16: 566-567).

The above methods are illustrative, and are not limited thereto.

For example, the amino acid corresponding to position 92 of the amino acid sequence of SEQ ID NO: 1 of the present disclosure may be any amino acid at positions 90 to 100 in an amino acid sequence of a homologous enzyme of SEQ ID NO: 1. In this case, a variant polypeptide in which tyrosine, the amino acid corresponding to position 92 of the amino acid sequence of SEQ ID NO: 1, is substituted with tryptophan, may be a variant polypeptide in which tyrosine corresponding to any one of positions 90 to 100 of the amino acid sequence of the homologous enzyme of SEQ ID NO: 1 is substituted with tryptophan. For another example, the amino acid corresponding to position 380 of the amino acid sequence of SEQ ID NO: 1 of the present disclosure may be any amino acid at positions 360 to 381 in the amino acid sequence of the homologous enzyme of SEQ ID NO: 1, and the amino acid corresponding to position 381 of the amino acid sequence of SEQ ID NO: 1 of the present disclosure may be any amino acid at positions 361 to 382 in the amino acid sequence of the homologous enzyme of SEQ ID NO: 1, but are not limited thereto.

In the present disclosure, with regard to the amino acid sequence, it is obvious that a polypeptide or protein "comprising," "consisting of," or "having" an amino acid sequence represented by a specific sequence number may also comprise a polypeptide or protein in which some amino acid(s) are deleted, modified, substituted, or added, as long as it has identical or corresponding activity to that of the polypeptide or protein of the corresponding sequence number. For example, the polypeptide or protein may also comprise a polypeptide or protein having additions or deletions of amino acid(s), which do not alter the function of the protein, inside, upstream, or downstream (N-terminus or C-terminus) of the polypeptide or protein, naturally occurring mutations, silent mutations, or conservative substitutions thereof, as long as it has identical or corresponding activity.

Further, for example, a polypeptide or protein conjugated with an N-terminal signal (or leader) sequence that is co-translationally or post-translationally involved in the protein (polypeptide) translocation, or a polypeptide or protein conjugated with other sequences or linkers for the purposes of identification, purification, or synthesis of the polypeptide or protein, may also be comprised within the scope of the polypeptide or protein of the amino acid sequence represented by the specific sequence number.

As used herein, the term "conservative substitution" means the substitution of one amino acid with another amino acid having similar structural and/or chemical properties. Such an amino acid substitution may generally occur based on similarity in the polarity, charge, solubility, hydrophobicity, hydrophilicity, and/or amphipathic nature of the residues. Usually, conservative substitution has minimal effect on the activity of a polypeptide or protein.

As used herein, the term "variant" or "variant polypeptide" refers to a polypeptide having a sequence different from the parent sequence of the polypeptide due to the presence of conservative substitution and/or modification (such as substitution, addition, or deletion) in one or more amino acids of the parent sequence. The functions or properties of the variant polypeptide may be increased, unchanged, or decreased as compared to that of the native polypeptide. For example, some variant polypeptides may comprise variant polypeptides in which one or more regions such as an N-terminal leader sequence or a transmembrane domain have been removed by substituting any one or more of the amino acids corresponding to positions 92, 208, 380, and 381 of the amino acid sequence of SEQ ID NO: 1 with different amino acids. Further, for example, other variant polypeptides may comprise variants in which a portion of the N-and/or C-terminus has been additionally removed from the mature protein. The term "variant polypeptide" may be used interchangeably with terms such as modification, modified protein, mutant, mutein, divergent, variant, etc., and is not limited thereto as long as it is a term used with the meaning of variation.

Further, the variant polypeptide may comprise deletions or additions of amino acids that have minimal effect on the properties and secondary structure of the polypeptide. For example, the polypeptide may be conjugated with a polypeptide N-terminal signal (or leader) sequence that is co-translationally or post-translationally involved in the protein translocation. Further, the polypeptide may be conjugated with other sequences or linkers so as to identify, purify, or synthesize the polypeptide.

The variant polypeptide of the present disclosure may be, but is not limited to, in an isolated form.

As used herein, the term "isolated" refers to a substance present in an environment that does not occur naturally or that is present in a form that does not exist naturally. This comprises substantial isolation of a substance (sequence or nucleic acid) from a substance which is naturally associated and found in nature, for example, at least one other component having a sequence or nucleic acid.

In the present disclosure, the variant polypeptide may be, but is not limited to, a non-naturally occurring polypeptide, i.e., a polypeptide that in itself does not exist in nature.

The variant polypeptide of the present disclosure may comprise biologically active fragments of the variant polypeptide.

In the present disclosure, in relation to an amino acid or base (nucleic acid) sequence, the term "biologically active fragments or fragments" may refer to "functional fragments". The "functional fragment" may also refer to an active fragment, and refers to a polypeptide that comprises fewer amino acids than a full-length protein but possesses at least one biological activity of the corresponding full-length protein. For example, the functional fragment of the enzyme may comprise a catalytic site of the enzyme.

As used herein, the term "different amino acid" is not limited as long as it is an amino acid different from the amino acid before substitution.

The variant polypeptide of the present disclosure may have substitutions of any one or more of the amino acids corresponding to positions 92, 208, 380, and 381 of the amino acid sequence of SEQ ID NO: 1 with different amino acids.

The variant polypeptide of the present disclosure may comprise substitutions of any one or more of the amino acids corresponding to positions 92, 208, 380, and 381 from the N-terminus of the amino acid sequence of SEQ ID NO: 1 with different amino acids, in an amino acid sequence having at least 70%, 75%, 80%, 85%, 90%, 95%, 96%, 97%, 98%, 99%, 99.5%, 99.7%, or 99.9% or more homology or identity to the amino acid sequence represented by SEQ ID NO: 1. It is also obvious that any variant having an amino acid sequence with deletion, substitution, conservative substitution, or addition in part of the sequence may also fall within the scope of the present disclosure, as long as the amino acid sequence comprises the amino acid substitutions while having homology or identity described above and exhibiting efficacy corresponding to that of the variant of the present disclosure.

In the present disclosure, when expressed as "the amino acid corresponding to position 92 of the amino acid sequence of SEQ ID NO: 1 is substituted with a different amino acid" and/or "the amino acid corresponding to position 208 of the amino acid sequence of SEQ ID NO: 1 is substituted with a different amino acid", it may be a substitution with an amino acid excluding tyrosine, when expressed as "the amino acid corresponding to position 380 of the amino acid sequence of SEQ ID NO: 1 is substituted with a different amino acid", it may be a substitution with an amino acid excluding phenylalanine, and when expressed as "the amino acid corresponding to position 381 of the amino acid sequence of SEQ ID NO: 1 is substituted with a different amino acid", it may be a substitution with an amino acid excluding glycine, and it may be a substitution with any one of alanine, glutamic acid (glutamate), phenylalanine, glycine, arginine, aspartate, cysteine, asparagine, glutamine, histidine, proline, serine, tyrosine, isoleucine, lysine, tryptophan, valine, methionine, threonine, or leucine, but are not limited thereto.

In one embodiment, the variant polypeptide of the present disclosure may comprise any one or more of the following substitutions: a substitution of the amino acid corresponding to position 92 of the amino acid sequence of SEQ ID NO: 1 with tryptophan; a substitution of the amino acid corresponding to position 208 with alanine or methionine; a substitution of the amino acid corresponding to position 380 with histidine, threonine, alanine, or glycine; and a substitution of the amino acid corresponding to position 381 with phenylalanine, serine, alanine, or asparagine.

For example, the variant polypeptide comprising the substitution of the amino acid corresponding to position 92 of the amino acid sequence of SEQ ID NO: 1 of the present disclosure with tryptophan may comprise an amino acid sequence having at least 70% or more, 75% or more, 80% or more, 85% or more, 90% or more, 95% or more, 96% or more, 97% or more, 98% or more, or 99% or more; and less than 100% homology or identity to SEQ ID NO: 1 while the amino acid corresponding to position 92 of the amino acid sequence represented by SEQ ID NO: 1 is fixed with tryptophan. Further, it is apparent that a variant polypeptide having an amino acid sequence having deletion, modification, substitution, conservative substitution, or addition of some sequence also falls within the scope of the present disclosure as long as the amino acid sequence has efficacy corresponding to that of the variant polypeptide of the present disclosure while having such homology or identity.

The variant polypeptide of the present disclosure may comprise substitution(s) of one to four amino acids, specifically, any one or more, two or more, three or more, or four amino acids of the amino acids corresponding to positions 92, 208, 380, and 381 of the amino acid sequence of SEQ ID NO: 1 with different amino acid(s).

The variant polypeptide of the present disclosure may comprise any variant polypeptide without limitation as long as it comprises substitutions of any one or more of the amino acids corresponding to positions 92, 208, 380, and 381 of the amino acid sequence of SEQ ID NO: 1 described above.

In one embodiment, the variant polypeptide may further comprise a substitution of the amino acid corresponding to position 380 of the amino acid sequence of SEQ ID NO: 1 with glycine, in addition to comprising any one or more of the following: the substitution of the amino acid corresponding to position 92 of the amino acid sequence of SEQ ID NO: 1 with tryptophan; the substitution of the amino acid corresponding to position 208 with alanine or methionine; and the substitution of the amino acid corresponding to position 381 with phenylalanine, serine, alanine, or asparagine.

In one embodiment, the variant polypeptide may comprise the substitution of the amino acid corresponding to position 92 of the amino acid sequence of SEQ ID NO: 1 with tryptophan; the substitution of the amino acid corresponding to position 208 with alanine or methionine; the substitution of the amino acid corresponding to position 380 with histidine, threonine, or alanine; the substitution of the amino acid corresponding to position 381 with phenylalanine, serine, alanine, or asparagine; or the substitution of the amino acid corresponding to position 380 with histidine, threonine, or glycine, and the substitution of the amino acid corresponding to position 381 with phenylalanine, serine, alanine, or asparagine.

In one embodiment, the variant polypeptide may further comprise a substitution of the amino acid corresponding to position 380 of the amino acid sequence of SEQ ID NO: 1 with histidine, glycine, threonine, or alanine, in addition to the substitution of the amino acid corresponding to position 381 of the amino acid sequence of SEQ ID NO: 1 with phenylalanine, serine, alanine, or asparagine. Further, the variant polypeptide may further comprise a substitution of the amino acid corresponding to position 92 of the amino acid sequence of SEQ ID NO: 1 with tryptophan, a substitution of the amino acid corresponding to position 208 with alanine, or a combination thereof, but is not limited thereto. In this regard, in one embodiment, the variant polypeptide may comprise a substitution of the amino acid corresponding to position 380 of the amino acid sequence of SEQ ID NO: 1 with alanine, and a substitution of the amino acid corresponding to position 381 with alanine; a substitution of the amino acid corresponding to position 380 of the amino acid sequence of SEQ ID NO: 1 with threonine, and a substitution of the amino acid corresponding to position 381 with phenylalanine; or a substitution of the amino acid corresponding to position 380 of the amino acid sequence of SEQ ID NO: 1 with threonine, and a substitution of the amino acid corresponding to position 381 with serine, but is not limited thereto.

In one embodiment, the variant polypeptide may comprise a substitution of the amino acid corresponding to position 208 with alanine, a substitution of the amino acid corresponding to position 380 with threonine or alanine, and a substitution of the amino acid corresponding to position 381 with phenylalanine, serine, or alanine; a substitution of the amino acid corresponding to position 92 with tryptophan, a substitution of the amino acid corresponding to position 380 with threonine or alanine, and a substitution of the amino acid corresponding to position 381 with phenylalanine, serine, or alanine; a substitution of the amino acid corresponding to position 92 with tryptophan, and a substitution of the amino acid corresponding to position 208 with alanine; or a substitution of the amino acid corresponding to position 92 with tryptophan, a substitution of the amino acid corresponding to position 208 with alanine, a substitution of the amino acid corresponding to position 380 with threonine or alanine, and a substitution of the amino acid corresponding to position 381 with phenylalanine, serine, or alanine.

In one embodiment of the above-described embodiments, the variant polypeptide may comprise a substitution of the amino acid corresponding to position 380 of the amino acid sequence of SEQ ID NO: 1 with histidine, and a substitution of the amino acid corresponding to position 381 with phenylalanine; a substitution of the amino acid corresponding to position 380 of the amino acid sequence of SEQ ID NO: 1 with histidine, and a substitution of the amino acid corresponding to position 381 with asparagine; a substitution of the amino acid corresponding to position 380 of the amino acid sequence of SEQ ID NO: 1 with histidine, and a substitution of the amino acid corresponding to position 381 with serine; a substitution of the amino acid corresponding to position 380 of the amino acid sequence of SEQ ID NO: 1 with histidine, and a substitution of the amino acid corresponding to position 381 with alanine; a substitution of the amino acid corresponding to position 380 of the amino acid sequence of SEQ ID NO: 1 with glycine, and a substitution of the amino acid corresponding to position 381 with phenylalanine; a substitution of the amino acid corresponding to position 380 of the amino acid sequence of SEQ ID NO: 1 with glycine, and a substitution of the amino acid corresponding to position 381 with asparagine; a substitution of the amino acid corresponding to position 380 of the amino acid sequence of SEQ ID NO: 1 with glycine, and a substitution of the amino acid corresponding to position 381 with serine; a substitution of the amino acid corresponding to position 380 of the amino acid sequence of SEQ ID NO: 1 with glycine, and a substitution of the amino acid corresponding to position 381 with alanine; a substitution of the amino acid corresponding to position 380 of the amino acid sequence of SEQ ID NO: 1 with threonine, and a substitution of the amino acid corresponding to position 381 with phenylalanine; a substitution of the amino acid corresponding to position 380 of the amino acid sequence of SEQ ID NO: 1 with threonine, and a substitution of the amino acid corresponding to position 381 with asparagine; a substitution of the amino acid corresponding to position 380 of the amino acid sequence of SEQ ID NO: 1 with threonine, and a substitution of the amino acid corresponding to position 381 with serine; a substitution of the amino acid corresponding to position 380 of the amino acid sequence of SEQ ID NO: 1 with threonine, and a substitution of the amino acid corresponding to position 381 with alanine; a substitution of the amino acid corresponding to position 380 of the amino acid sequence of SEQ ID NO: 1 with alanine, and a substitution of the amino acid corresponding to position 381 with phenylalanine; a substitution of the amino acid corresponding to position 380 of the amino acid sequence of SEQ ID NO: 1 with alanine, and a substitution of the amino acid corresponding to position 381 with asparagine; a substitution of the amino acid corresponding to position 380 of the amino acid sequence of SEQ ID NO: 1 with alanine, and a substitution of the amino acid corresponding to position 381 with serine; a substitution of the amino acid corresponding to position 380 of the amino acid sequence of SEQ ID NO: 1 with alanine, and a substitution of the amino acid corresponding to position 381 with alanine; a substitution of the amino acid corresponding to position 208 of the amino acid sequence of SEQ ID NO: 1 with alanine, a substitution of the amino acid corresponding to position 380 with alanine, and a substitution of the amino acid corresponding to position 381 with alanine; a substitution of the amino acid corresponding to position 208 of the amino acid sequence of SEQ ID NO: 1 with alanine, a substitution of the amino acid corresponding to position 380 with threonine, and a substitution of the amino acid corresponding to position 381 with phenylalanine; a substitution of the amino acid corresponding to position 208 of the amino acid sequence of SEQ ID NO: 1 with alanine, a substitution of the amino acid corresponding to position 380 with threonine, and a substitution of the amino acid corresponding to position 381 with serine; a substitution of the amino acid corresponding to position 92 of the amino acid sequence of SEQ ID NO: 1 with tryptophan, a substitution of the amino acid corresponding to position 380 with alanine, and a substitution of the amino acid corresponding to position 381 with alanine; a substitution of the amino acid corresponding to position 92 of the amino acid sequence of SEQ ID NO: 1 with tryptophan, a substitution of the amino acid corresponding to position 380 with threonine, and a substitution of the amino acid corresponding to position 381 with phenylalanine; a substitution of the amino acid corresponding to position 92 of the amino acid sequence of SEQ ID NO: 1 with tryptophan, a substitution of the amino acid corresponding to position 380 with threonine, and a substitution of the amino acid corresponding to position 381 with serine; a substitution of the amino acid corresponding to position 92 of the amino acid sequence of SEQ ID NO: 1 with tryptophan, and a substitution of the amino acid corresponding to position 208 with alanine; a substitution of the amino acid corresponding to position 92 of the amino acid sequence of SEQ ID NO: 1 with tryptophan, a substitution of the amino acid corresponding to position 208 with alanine, a substitution of the amino acid corresponding to position 380 with alanine, and a substitution of the amino acid corresponding to position 381 with alanine; a substitution of the amino acid corresponding to position 92 of the amino acid sequence of SEQ ID NO: 1 with tryptophan, a substitution of the amino acid corresponding to position 208 with alanine, a substitution of the amino acid corresponding to position 380 with threonine, and a substitution of the amino acid corresponding to position 381 with phenylalanine; or a substitution of the amino acid corresponding to position 92 with tryptophan, a substitution of the amino acid corresponding to position 208 with alanine, a substitution of the amino acid corresponding to position 380 with threonine, and a substitution of the amino acid corresponding to position 381 with serine, but is not limited thereto.

In one embodiment of the above-described embodiments, the variant polypeptide may consist of an amino acid sequence of SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 65, SEQ ID NO: 67, SEQ ID NO: 69, SEQ ID NO: 71, or SEQ ID NO: 73.

Specifically, the variant polypeptide of the present disclosure may have or comprise an amino acid sequence having at least 70%, 70%, 80%, 85%, 90%, 95%, 96%, 97%, 98%, or 99% or more homology or identity to the sequence numbers, or may consist of the amino acid sequence, or may essentially consist of the amino acid sequence.

In one embodiment, among the variant polypeptides of the present disclosure, the variant polypeptide comprising the single substitution of the amino acid corresponding to position 92 of the amino acid sequence of SEQ ID NO: 1 with tryptophan may be SEQ ID NO: 3; the variant polypeptide comprising the single substitution of the amino acid corresponding to position 208 of the amino acid sequence of SEQ ID NO: 1 with alanine or methionine may be SEQ ID NO: 5 or SEQ ID NO: 7, respectively; the variant polypeptide comprising the single substitution of the amino acid corresponding to position 380 of the amino acid sequence of SEQ ID NO: 1 with histidine, threonine, or alanine may be SEQ ID NO: 9, SEQ ID NO: 11, or SEQ ID NO: 13, respectively; the variant polypeptide comprising the substitution of the amino acid corresponding to position 381 of the amino acid sequence of SEQ ID NO: 1 with phenylalanine, serine, alanine, or asparagine may be SEQ ID NO: 15, SEQ ID NO: 17, SEQ ID NO: 19, or SEQ ID NO: 21, respectively; the variant polypeptide comprising the double substitution comprising the substitution of the amino acid corresponding to position 380 of the amino acid sequence of SEQ ID NO: 1 with histidine, and the substitution of the amino acid corresponding to position 381 with phenylalanine, asparagine, serine, or alanine may be SEQ ID NO: 23, SEQ ID NO: 25, SEQ ID NO: 27, or SEQ ID NO: 29, respectively; the variant polypeptide comprising the double substitution comprising the substitution of the amino acid corresponding to position 380 of the amino acid sequence of SEQ ID NO: 1 with glycine, and the substitution of the amino acid corresponding to position 381 with phenylalanine, asparagine, serine, or alanine may be SEQ ID NO: 31, SEQ ID NO: 33, SEQ ID NO: 35, or SEQ ID NO: 37, respectively; the variant polypeptide comprising the double substitution comprising the substitution of the amino acid corresponding to position 380 of the amino acid sequence of SEQ ID NO: 1 with threonine, and the substitution of the amino acid corresponding to position 381 with phenylalanine, asparagine, serine, or alanine may be SEQ ID NO: 39, SEQ ID NO: 41, SEQ ID NO: 43, or SEQ ID NO: 45, respectively; the variant polypeptide comprising the double substitution comprising the substitution of the amino acid corresponding to position 380 of the amino acid sequence of SEQ ID NO: 1 with alanine, and the substitution of the amino acid corresponding to position 381 with phenylalanine, asparagine, serine, or alanine may be SEQ ID NO: 47, SEQ ID NO: 49, SEQ ID NO: 51, or SEQ ID NO: 53, respectively; the variant polypeptide comprising the substitution of the amino acid corresponding to position 208 of the amino acid sequence of SEQ ID NO: 1 with alanine, the substitution of the amino acid corresponding to position 380 with alanine, and the substitution of the amino acid corresponding to position 381 with alanine may be SEQ ID NO: 55; the variant polypeptide comprising the substitution of the amino acid corresponding to position 208 of the amino acid sequence of SEQ ID NO: 1 with alanine, the substitution of the amino acid corresponding to position 380 with threonine, and the substitution of the amino acid corresponding to position 381 with phenylalanine may be SEQ ID NO: 57; the variant polypeptide comprising the substitution of the amino acid corresponding to position 208 of the amino acid sequence of SEQ ID NO: 1 with alanine, the substitution of the amino acid corresponding to position 380 with threonine, and the substitution of the amino acid corresponding to position 381 with serine may be SEQ ID NO: 59; the variant polypeptide comprising the substitution of the amino acid corresponding to position 92 of the amino acid sequence of SEQ ID NO: 1 with tryptophan, the substitution of the amino acid corresponding to position 380 with alanine, and the substitution of the amino acid corresponding to position 381 with alanine may be SEQ ID NO: 61; the variant polypeptide comprising the substitution of the amino acid corresponding to position 92 of the amino acid sequence of SEQ ID NO: 1 with tryptophan, the substitution of the amino acid corresponding to position 380 with threonine, and the substitution of the amino acid corresponding to position 381 with phenylalanine may be SEQ ID NO: 63; the variant polypeptide comprising the substitution of the amino acid corresponding to position 92 of the amino acid sequence of SEQ ID NO: 1 with tryptophan, the substitution of the amino acid corresponding to position 380 with threonine, and the substitution of the amino acid corresponding to position 381 with serine may be SEQ ID NO: 65; the variant polypeptide comprising the substitution of the amino acid corresponding to position 92 of the amino acid sequence of SEQ ID NO: 1 with tryptophan, and the substitution of the amino acid corresponding to position 208 with alanine may be SEQ ID NO: 67; the variant polypeptide comprising the substitution of the amino acid corresponding to position 92 of the amino acid sequence of SEQ ID NO: 1 with tryptophan, and the substitutions of the amino acids corresponding to positions 208, 380, and 381 with alanine may be SEQ ID NO: 69; the variant polypeptide comprising the substitution of the amino acid corresponding to position 92 of the amino acid sequence of SEQ ID NO: 1 with tryptophan, the substitution of the amino acid corresponding to position 208 with alanine, the substitution of the amino acid corresponding to position 380 with threonine, and the substitution of the amino acid corresponding to position 381 with phenylalanine may be SEQ ID NO: 71; and the variant polypeptide comprising the substitution of the amino acid corresponding to position 92 of the amino acid sequence of SEQ ID NO: 1 with tryptophan, the substitution of the amino acid corresponding to position 208 with alanine, the substitution of the amino acid corresponding to position 380 with threonine, and the substitution of the amino acid corresponding to position 381 with serine may be SEQ ID NO: 73, but are not limited thereto.

In one embodiment, the variant polypeptide of the present disclosure may comprise any one or more of the following: the substitution of tyrosine, which is the amino acid corresponding to position 92 of the amino acid sequence of SEQ ID NO: 1, with tryptophan; the substitution of tyrosine, which is the amino acid corresponding to position 208, with alanine or methionine; the substitution of phenylalanine, which is the amino acid corresponding to position 380, with histidine, threonine, or alanine; and the substitution of glycine, which is the amino acid corresponding to position 381, with phenylalanine, serine, alanine, or asparagine.

In one embodiment of the above-described embodiments, the variant polypeptide may further comprise the substitution of phenylalanine, which is the amino acid corresponding to position 380 of the amino acid sequence of SEQ ID NO: 1, with glycine, while comprising the substitution of tyrosine, which is the amino acid corresponding to position 92 of the amino acid sequence of SEQ ID NO: 1, with tryptophan; the substitution of tyrosine, which is the amino acid corresponding to position 208, with alanine or methionine; and the substitution of glycine, which is the amino acid corresponding to position 381, with phenylalanine, serine, alanine, or asparagine, but is not limited thereto.

Another aspect of the present disclosure provides a polynucleotide encoding the variant polypeptide of the present disclosure.

As used herein, the term "polynucleotide" is a DNA or RNA strand having a certain length or more as a polymer of nucleotides in which nucleotide monomers are connected in a long chain by covalent bonds, and more specifically, refers to a polynucleotide fragment encoding the variant polypeptide.

The polynucleotide of the present disclosure may comprise any polynucleotide sequence without limitation, as long as it is a polynucleotide sequence encoding the variant polypeptide of the present disclosure. The polynucleotide may be prepared based on codon information known in the art, but is not limited thereto. For example, the polynucleotide may comprise nucleotide sequences encoding amino acid sequences represented by any one or more of SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 65, SEQ ID NO: 67, SEQ ID NO: 69, SEQ ID NO: 71, and SEQ ID NO: 73. For example, the polynucleotide of the present disclosure may have, comprise, consist of, or essentially consist of any one or more sequences of SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44 or SEQ ID NO: 128, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68, SEQ ID NO: 70, SEQ ID NO: 72, and SEQ ID NO: 74.

The polynucleotide of the present disclosure may undergo various modifications in the coding region within a scope that does not change the amino acid sequence of the variant polypeptide of the present disclosure in consideration of codon degeneracy or codons preferred in organisms that are intended to express the variant polypeptide of the present disclosure. Therefore, it is obvious that, due to codon degeneracy, a polynucleotide to be translated into a polypeptide consisting of the amino acid sequence of the variant polypeptide of the present disclosure or a polypeptide having homology or identity thereto may also be comprised. For example, the polynucleotide of the present disclosure may be a nucleotide sequence of SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44 or SEQ ID NO: 128, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68, SEQ ID NO: 70, SEQ ID NO: 72, or SEQ ID NO: 74, or a degenerated sequence thereof. For example, SEQ ID NO: 2 and SEQ ID NO: 44 may be codon-optimized for a microorganism of the genus *Corynebacterium*, and SEQ ID NO: 127 and SEQ ID NO: 128 may be codon-optimized for a microorganism of the genus *Escherichia* (e.g., *E. coli*).

For another example, the polynucleotide of the present disclosure may have or comprise a nucleotide sequence having 70% or more, 75% or more, 80% or more, 85% or more, 90% or more, 95% or more, 96% or more, 97% or more, 98% or more, or 99% or more homology or identity to any one or more sequences of SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44 or SEQ ID NO: 128, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68, SEQ ID NO: 70, SEQ ID NO: 72, and SEQ ID NO: 74, or may consist of or essentially consist of the nucleotide sequence, but is not limited thereto.

For example, the polynucleotide of the present disclosure may comprise a nucleotide sequence having any one or more fixations of a fixation of the codon encoding the amino acid corresponding to position 92 of SEQ ID NO: 1 with one of the codons encoding tryptophan; a fixation of the codon encoding the amino acid corresponding to position 208 with one of the codons encoding alanine or methionine; a fixation of the codon encoding the amino acid corresponding to position 380 with one of the codons encoding histidine, threonine, or alanine; and a fixation of the codon encoding the amino acid corresponding to position 381 with one of the codons encoding phenylalanine, serine, alanine, or asparagine.

In one embodiment, the polynucleotide sequences encoding SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 65, SEQ ID NO: 67, SEQ ID NO: 69, SEQ ID NO: 71, and SEQ ID NO: 73 may be SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44 or SEQ ID NO: 128 (both sequences may encode SEQ ID NO: 43), SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68, SEQ ID NO: 70, SEQ ID NO: 72, and SEQ ID NO: 74, but are not limited thereto.

Further, the polynucleotide of the present disclosure may comprise a probe that may be prepared from a known gene sequence, for example, may comprise any sequence without limitation as long as it is a sequence that is able to hybridize with a complementary sequence to the entirety or part of the polynucleotide sequence of the present disclosure under stringent conditions and that encodes the variant polypeptide of the present disclosure. The stringent conditions are as explained above.

As used herein, the term "identity" or "homology" refers to the relevance between two given amino acid sequences or nucleotide sequences and may be expressed as a percentage. In the present disclosure, "homology" and "identity" may often be used interchangeably.

The sequence homology or identity of conserved polynucleotide or polypeptide may be determined by standard alignment algorithms and may be used with a default gap penalty established by the program being used.

Whether or not any two polynucleotide or polypeptide sequences have homology, similarity, or identity may be determined using known computer algorithms such as the "FASTA" program, for example, using default parameters as in Pearson et al (1988) [Proc. Natl. Acad. Sci. USA 85]: 2444. Alternatively, it may be determined by comparing sequence information using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) or by using the GAP computer program, such as the Smith-Waterman algorithm (Smith and Waterman, Adv. Appl. Math (1981) 2:482) (comprising GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO et al.](1988) SIAM J Applied Math 48: 1073). For example, BLAST or ClustalW of the National Center for Biotechnology Information may be used to determine the homology, similarity, or identity.

Further, whether any two polynucleotide sequences have homology, similarity, or identity may be determined by Southern hybridization experiments under appropriate hybridization conditions, and the appropriate hybridization conditions may be determined by a method well known to those skilled in the art (e.g., J. Sambrook et al., Molecular Cloning, A Laboratory Manual; F.M. Ausubel et al., Current Protocols in Molecular Biology, John Wiley & Sons, Inc., New York), but are not limited thereto. For example, homologous or identical polynucleotide sequences may generally hybridize along the entirety of the sequence or at least about 50%, 60%, 70%, 80%, or 90% of the full-length of the sequence under stringent conditions.

As used herein, the term "stringent conditions" refers to conditions under which specific hybridization between polynucleotides is allowed. Such conditions are specifically described in the literature (see Sambrook et al., supra, 9.50-9.51, 11.7-11.8). For example, the stringent conditions may comprise conditions under which polynucleotides having high homology or identity, polynucleotides having 60% or more, 70% or more, 75% or more, 80% or more, 85% or more, 90% or more, 95% or more, 96% or more, 97% or more, 98% or more, or 99% or more homology or identity are hybridized with each other, and polynucleotides having homology or identity lower than the above are not hybridized with each other, or washing conditions of the common Southern hybridization, i.e., washing once, specifically, twice or three times at a salt concentration and a temperature corresponding to 60°C, 1XSSC, 0.1% SDS, specifically 60°C, 0.1XSSC, 0.1% SDS, and more specifically 68°C, 0.1XSSC, 0.1% SDS.

The hybridization may occur between nucleotides having bases of sequences complementary to each other, but hybridized polynucleotides may comprise some mismatches between bases depending on the stringency of hybridization. The term "complementary" is used to describe the relation between nucleotide bases capable of being hybridized with each other. For example, with regard to DNA, adenine is complementary to thymine and cytosine is complementary to guanine. Hence, the polynucleotide of the present disclosure may also comprise substantially similar nucleotide sequences as well as isolated nucleic acid fragments that are complementary to the entire sequence.

For example, a polynucleotide having homology or identity to the polynucleotide of the present disclosure may be detected by hybridization at a Tm value of 55°C. Further, the Tm value may be 60°C, 63°C, or 65°C, but is not limited thereto, and may be appropriately adjusted by those skilled in the art.

The appropriate stringency to hybridize the polynucleotide depends on the length and degree of complementarity of the polynucleotide, and the variables are well known in the art (e.g., J. Sambrook et al., supra).

Still another aspect of the present disclosure provides a vector comprising the polynucleotide of the present disclosure. The vector may be an expression vector for expressing the polynucleotide in a microorganism, but is not limited thereto.

As used herein, the term "vector" means a DNA construct for delivering a desired polynucleotide into an appropriate host or host cell.

For example, the vector may comprise a nucleotide sequence of a polynucleotide encoding a desired polypeptide operably linked to a suitable expression regulatory region (regulatory sequence) so as to express the desired polypeptide in a suitable host cell. The expression regulatory region may comprise a promoter capable of initiating transcription, any operator sequence for regulating transcription, a sequence encoding a suitable mRNA ribosome-binding site and a sequence for regulating termination of transcription and translation. Once transformed into a suitable host cell (microorganism), the vector may replicate or function independently from the host genome, or may integrate into the genome thereof to replicate or function.

For example, the vector of the present disclosure may comprise a sequence for inserting the desired polynucleotide into the chromosome. Insertion of the polynucleotide into the chromosome using the vector may be performed by any method known in the art, for example, homologous recombination, but is not limited thereto.

The vector used in the present disclosure is not particularly limited, but any vector known in the art may be used. Examples of vectors commonly used may comprise natural or recombinant plasmids, cosmids, viruses, and bacteriophages. For example, pWE15, M13, MBL3, MBL4, IXII, ASHII, APII, t10, t11, Charon4A, Charon21A, etc. may be used as a phage vector or a cosmid vector, and pDZ system, pDC system, pBR system, pUC system, pBluescript II system, pGEM system, pTZ system, pCL system, pET system, etc. may be used as a plasmid vector. For example, pDZ, pDC, pDCM2, pACYC177, pACYC184, pCL, pECCG117, pUC19, pBR322, pMW118, pCC1BAC, pCES208, pET-28a vector, etc. may be used.

The vector may further comprise a selection marker to confirm transformation into a host cell, or furthermore, insertion into the chromosome in the host cell. The selection marker is for selecting the cells transformed with the vector, or for confirming insertion of the desired polynucleotide into the chromosome, and markers that provide selectable phenotypes, such as drug resistance, auxotrophy, resistance to cytotoxic agents, or expression of surface polypeptides, may be used. Only cells expressing the selection marker are able to survive or exhibit different phenotypes under an environment treated with the selective agent, and thus the transformed cells may be selected.

As used herein, the term "transformation" means that a desired polynucleotide or a vector comprising the same is introduced into a host cell (also comprising a microorganism) to change the genetic trait of the host cell. The transformed polynucleotide may be inserted into the chromosome of the host cell or may be located outside the chromosome. Further, the polynucleotide may comprise DNA or RNA. The polynucleotide may be introduced in an appropriate form depending on the purpose of introduction. For example, the polynucleotide for expressing the desired polypeptide may be introduced into a host cell (microorganism) in the form of an expression cassette, which is a gene construct comprising all elements required for self-expression. The expression cassette may usually comprise a promoter operably linked to a coding sequence of the desired polypeptide, a transcription termination signal, a ribosome binding site, and a translation termination signal. The expression cassette may be in the form of an expression vector capable of self-replicating. Further, the polynucleotide may be introduced into a host cell (microorganism) in its own form and operably linked to a sequence required for expression in the host cell (microorganism), but is not limited thereto.

As used herein, the term "operably linked" means a configuration in which a regulatory sequence is placed at an appropriate position such that the regulatory sequence directs expression of the coding sequence. Thus, the term "operably linked" comprises an attachment or linking between a regulatory region of a functional domain having a known or desired activity, such as a promoter, a terminator, a signal sequence, or an enhancer, and a target (gene or polypeptide) such that the expression, secretion, or function of the target may be regulated in accordance with the known or desired activity. For example, the term means that the polynucleotide sequence is functionally linked to a promoter sequence that initiates and mediates transcription of the polynucleotide encoding the polypeptide.

As used herein, the term "genetic recombination" refers to a natural or artificial process in which elements of a gene, such as DNA or RNA, are changed from their original sequence during the disassembly and reassembly process.

As used herein, the term "recombinant gene" means a gene having a new genetic configuration resulting from genetic recombination, such as chemical synthesis or genetic engineering techniques, etc. In the present disclosure, the terms "recombinant gene," "recombinant DNA," and "recombinant polynucleotide" may be used interchangeably with each other. For example, the recombinant gene may comprise an artificial combination of nucleic acid fragments, such as regulatory sequences, which are not found together in nature.

Still another aspect of the present disclosure provides a microorganism comprising any one or more of the variant polypeptide of the present disclosure, the polynucleotide encoding the variant polypeptide, and the vector comprising the polynucleotide.

The variant polypeptide, polynucleotide, and vector are as explained in other aspects.

As used herein, the term "microorganism (or strain)" comprises all of wild-type microorganisms or naturally or artificially genetically modified prokaryotic or eukaryotic microorganisms, and it may be a microorganism in which a specific mechanism is weakened or enhanced due to insertion of a foreign gene or an activity enhancement or inactivation of an endogenous gene, etc., and it may be a microorganism comprising genetic modification for production of a desired polypeptide, protein, or product. As used herein, the term "microorganism", "strain", "host", and "host cell" may be used interchangeably.

As used herein, the term "recombinant microorganism" refers to a microorganism that has been genetically modified such that it exhibits a different genotype and/or phenotype compared to a naturally occurring microorganism (e.g., when the genetic modification affects the nucleotide sequence coding of the microorganism), and may comprise any progeny or potential progeny of the microorganism. As used herein, the term "recombinant microorganism", "genetically modified microorganism", "recombinant host cell", "recombinant cell" and "recombinant strain" may be used interchangeably.

For example, the microorganism of the present disclosure may be, but is not limited to, a microorganism modified to express the variant polypeptide of the present disclosure or a recombinant microorganism having the activity of the variant polypeptide of the present disclosure.

In one embodiment, the microorganism of the present disclosure may have the ability to produce any one or more of rebaudioside D and rebaudioside M.

As used herein, the term "microorganism having the ability to produce any one or more of rebaudioside D and rebaudioside M" refers to a prokaryotic or eukaryotic microorganism capable of producing rebaudioside D and/or M in a living organism, and the microorganisms may comprise both a microorganism that inherently has the ability to produce rebaudioside D and/or M, as well as a microorganism that does not inherently have the production ability but are endowed with the production ability. The ability to produce rebaudioside D and/or M may be endowed or enhanced by expression of the variant polypeptide of the present disclosure or by species improvement. The microorganism having the ability to produce any one or more of rebaudioside D and rebaudioside M may be used interchangeably with a "microorganism having the ability to produce rebaudioside D", a "microorganism having the ability to produce rebaudioside M", a "microorganism having the ability to produce rebaudioside D or M", and a "microorganism having the ability to produce rebaudioside D and M.

As used herein, the term "unmodified microorganism" does not exclude microorganisms comprising mutations that may naturally occur, and may be a wild-type microorganism or a natural microorganism itself or may be a microorganism before the trait is changed by genetic variation due to natural or artificial factors. In the present disclosure, the term "unmodified microorganism" may be used interchangeably with "microorganism before being modified", "unvaried microorganism", "parent microorganism", "parent strain", "wild-type microorganism", "reference microorganism", or "standard microorganism". In the present disclosure, the unmodified microorganism may mean a microorganism in which the variant polypeptide of the present disclosure is not introduced or has not yet been introduced; or a microorganism comprising the wild-type polypeptide, but is not limited thereto. Further, in the present disclosure, the unmodified microorganism may be a microorganism comprising the polypeptide consisting of SEQ ID NO: 1 or a polynucleotide encoding the same (e.g., the polynucleotide consisting of SEQ ID NO: 2), but is not limited thereto.

For example, the microorganism of the present disclosure comprises both a microorganism comprising the variant polypeptide sequence of the present disclosure through mutating the gene on the chromosome encoding the polypeptide comprising the parent sequence of the present disclosure; and/or a microorganism comprising the variant polypeptide of the present disclosure through introducing the vector comprising the polynucleotide encoding the variant polypeptide of the present disclosure, but is not limited thereto.

The microorganism of the present disclosure may comprise any microorganism comprising any one or more of the variant polypeptide of the present disclosure; the polynucleotide encoding the variant polypeptide; and the vector comprising the polynucleotide, and capable of producing any one or more of rebaudioside D and rebaudioside M. For example, the microorganism of the present disclosure may be a microorganism that is transformed with the vector comprising the polynucleotide encoding the variant polypeptide of the present disclosure to express the variant polypeptide of the present disclosure, thereby having the enhanced rebaudioside D and/or M-producing ability, but is not limited thereto.

The microorganism having the enhanced ability to produce any one or more of rebaudioside D and rebaudioside M may be, but is not limited to, a microorganism having an enhanced rebaudioside D and/or M-producing ability compared to a natural wild-type microorganism or an unmodified microorganism (e.g., a microorganism expressing a wild-type polypeptide or a polypeptide comprising a parent sequence; or a microorganism not expressing the variant polypeptide of the present disclosure).

For example, the microorganism having the enhanced ability to produce any one or more of rebaudioside D and rebaudioside M of the present disclosure may be, but is not limited to, a microorganism having the enhanced rebaudioside D and/or M-producing ability, as compared to a parent microorganism (parent strain) before modification or an unmodified microorganism (e.g., a microorganism expressing the wild-type polypeptide, the polypeptide comprising the parent sequence; or the polypeptide of SEQ ID NO: 1; or a microorganism not expressing the variant polypeptide of the present disclosure). For example, the parent microorganism (parent strain) before modification or the unmodified microorganism to be compared for the enhanced production ability may be, but is not limited to, *Corynebacterium ammoniagenes* ATCC6872.

For example, the microorganism having the enhanced production ability may have an enhanced rebaudioside D and/or M-producing ability of about 1% or more, specifically, about 5% or more, about 10% or more, about 20% or more, about 30% or more, about 50% or more, about 70% or more, about 90% or more, or about 100% or more (the upper limit is not particularly limited, but may be, for example, about 500% or less), as compared to that of the parent microorganism (parent strain) before modification or the unmodified microorganism, but the increased amount is not limited thereto as long as the production ability has an increased amount of a + value, as compared to the production ability of the parent microorganism (parent strain) before modification or the unmodified microorganism. In another example, the recombinant microorganism having the enhanced production ability may have an enhanced production ability of about 1.1 times or more, about 1.15 times or more, about 1.2 times or more, about 1.3 times or more, about 1.5 times or more, about 1.7 times or more, about 1.9 times or more, or about 2.0 times or more (the upper limit is not particularly limited, but may be, for example, about 10 times or less), as compared to that of the parent microorganism (parent strain) before modification or the unmodified microorganism, but is not limited thereto.

For example, the microorganism of the present disclosure may be either a prokaryotic cell or a eukaryotic cell, specifically a prokaryotic cell. The prokaryotic cell may comprise, for example, microorganisms of *Escherichia* sp., *Erwinia* sp., *Serratia* sp., *Providencia* sp., *Corynebacterium* sp., *Pseudomonas* sp., *Leptospira* sp., *Salmonella* sp., *Brevibacteria* sp., *Hypomononas* sp., *Chromobacterium* sp., and *Norcardia* sp., fungi, or yeasts, but is not limited thereto. Specifically, the prokaryotic cell may be a microorganism of *Escherichia* sp., *Corynebacterium* sp., *Leptospira* sp., and yeast. More specifically, the prokaryotic cell may be a microorganism of *Corynebacterium* sp. o*r Escherichia* sp.

With regard to the microorganism according to any one of the aforementioned specific embodiments, the microorganism of *Corynebacterium* sp. of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens.*

Specifically, the microorganism of the present disclosure may be *Corynebacterium ammoniagenes, Corynebacterium glutamicum,* or *Escherichia coli,* but is not limited thereto.

In one embodiment, the microorganism having the rebaudioside D and/or M-producing ability of the present disclosure may further comprise any one or more of a UGT-A polypeptide, a polynucleotide encoding the same and a vector comprising the same. The polypeptide and polynucleotide may be a wild-type or a variant, and the polypeptide may comprise any polypeptide without limitation to the type thereof, as long as it has UGT-A activity.

Specifically, the UGT-A of the present disclosure may have, comprise, consist of, or essentially consist of an amino acid sequence of SEQ ID NO: 129; or an amino acid sequence having at least 70%, 80%, 85%, 90%, 95%, 96%, 97%, 98%, or 99% or more homology or identity thereto.

Specifically, the polynucleotide encoding the UGT-A of the present disclosure may have, comprise, consist of, or essentially consist of a polynucleotide sequence of SEQ ID NO: 130; or a polynucleotide sequence having at least 70%, 80%, 85%, 90%, 95%, 96%, 97%, 98%, or 99% or more homology or identity thereto.

In one embodiment, the microorganism having the rebaudioside D and/or M-producing ability of the present disclosure may further comprise any one or more of a sucrose synthase, a polynucleotide encoding the same, and a vector comprising the same.

Specifically, the sucrose synthase of the present disclosure may have, comprise, consist of, or essentially consist of an amino acid sequence of SEQ ID NO: 131; or an amino acid sequence having at least 70%, 80%, 85%, 90%, 95%, 96%, 97%, 98%, or 99% or more homology or identity thereto.

Specifically, the polynucleotide encoding the sucrose synthase of the present disclosure may have, comprise, consist of, or essentially consist of a polynucleotide sequence of SEQ ID NO: 132; or a polynucleotide sequence having at least 70%, 70%, 80%, 85%, 90%, 95%, 96%, 97%, 98%, or 99% or more homology or identity thereto.

Still another aspect of the present disclosure provides a method of producing a steviol glycoside, the method comprising the step of culturing, in a medium, the microorganism comprising any one or more of the variant polypeptide of the present disclosure, the polynucleotide encoding the variant polypeptide, and the vector comprising the polynucleotide.

Specifically, the steviol glycoside may be any one or more of rebaudioside D and rebaudioside M.

The variant polypeptide, polynucleotide, vector, and microorganism are as explained in other aspects.

As used herein, the term "culturing" refers to growing a microorganism in appropriately adjusted environmental conditions. The culturing procedure may be performed according to appropriate media or culture conditions known in the art. Such a culturing process may be easily adjusted and used by a person skilled in the art according to the selected microorganism. Specifically, the culturing may be in a batch type, a continuous type, and/or a fed-batch type, but is not limited thereto.

As used herein, the "medium" refers to a mixture comprising, as main ingredients, nutrient materials required for culturing the microorganism, wherein the medium supplies nutrient materials comprising water essential for survival and growth, growth factors, etc. Specifically, as for the media and other culture conditions used for culturing the microorganism of the present disclosure, any medium that is commonly used for culturing microorganisms may be used without particular limitation. For example, the microorganism of the present disclosure may be cultured under aerobic conditions in a common medium comprising appropriate carbon sources, nitrogen sources, phosphorus sources, inorganic compounds, amino acids, and/or vitamins, while controlling the temperature, pH, etc. For example, a culture medium for the microorganisms of *Corynebacterium* sp. may be found in a literature ["Manual of Methods for General Bacteriology" by the American Society for Bacteriology (Washington D.C., USA, 1981)].

In the present disclosure, the carbon source may comprise carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; amino acids, such as glutamic acid, methionine, lysine, etc. In addition, natural organic nutrient sources may be used, such as starch hydrolysates, molasses, blackstrap molasses, rice bran, cassava, bagasse, and corn steep liquor, and specifically, carbohydrates, such as glucose and sterile pretreated molasses (i.e., molasses converted to reduced sugars) may be used, and appropriate amounts of various other carbon sources may be used without limitation. These carbon sources may be used alone or in a combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extracts, yeast extracts, malt extracts, corn steep liquor, casein hydrolysates, fishes or decomposition products thereof, defatted soybean cake or degradation products thereof, etc. may be used. These nitrogen sources may be used alone or in a combination of two or more thereof, but are not limited thereto.

The phosphate sources may comprise potassium phosphate monobasic, potassium phosphate dibasic, and sodium-comprising salts corresponding thereto. As for inorganic compounds, sodium chloride, calcium chloride, iron chloride, magnesium sulfate, iron sulfate, manganese sulfate, calcium carbonate, etc. may be used, and in addition, amino acids, vitamins, and/or suitable precursors may be comprised. These constituent ingredients or precursors may be added to the medium in a batch or continuous manner. However, the present disclosure is not limited thereto.

Further, the pH of the medium may be adjusted by adding compounds, such as ammonium hydroxide, potassium hydroxide, ammonia, phosphoric acid, sulfuric acid, etc., to the medium in an appropriate manner during the culturing of the microorganism of the present disclosure. In addition, an anti-foaming agent, such as a fatty acid polyglycol ester, may be added to suppress foam formation during culturing. In addition, oxygen or oxygen-comprising gas may be injected into the medium to maintain the aerobic state of the medium, or no gas may be injected or nitrogen, hydrogen or carbon dioxide gas may be injected to maintain the anaerobic or non-aerobic state of the medium, but is not limited thereto.

In one embodiment, the medium for the microorganism of the present disclosure may comprise a substrate for the synthesis of rebaudioside D and/or rebaudioside M. The medium may comprise any one or more of rebaudioside A, rebaudioside D, stevioside, nucleotide diphosphate-linked glucose, sucrose, and nucleotide diphosphate. Specifically, the medium for producing rebaudioside D may comprise rebaudioside A and nucleotide diphosphate-linked glucose; rebaudioside A, sucrose, and nucleotide diphosphate; or rebaudioside A, nucleotide diphosphate-linked glucose, sucrose, and nucleotide diphosphate. The medium may comprise stevioside and nucleotide diphosphate-linked glucose, instead of or in addition to rebaudioside A, and may comprise sucrose and nucleotide diphosphate, instead of or in addition to nucleotide diphosphate-linked glucose. The medium for producing rebaudioside M may comprise rebaudioside D and nucleotide diphosphate-linked glucose; or rebaudioside D, sucrose, and nucleotide diphosphate. Instead of rebaudioside D, the medium for producing rebaudioside D may be comprised. In one embodiment, the nucleotide diphosphate may be uridine diphosphate, but is not limited thereto.

In the culturing of the present disclosure, the culture temperature may be maintained at 20°C to 45°C, specifically 25°C to 40°C, and the culturing may be performed for about 10 hours to 160 hours, but is not limited thereto.

As used herein, the term "culture" refers to a culture liquid obtained by culturing a specific microorganism in a culture medium, a concentrated culture liquid, a dry product of the culture liquid, a culture filtrate, a concentrated culture filtrate, or a dry product of the culture filtrate, and the culture liquid refers to those comprising the specific microorganism, and the culture filtrate refers to those substantially not comprising the specific microorganism (which means that the specific microorganism to be separated by filtration, etc. are substantially excluded, which does not mean that the microorganism is completely excluded from the filtrate.). The formulation of the culture is not limited, but it may be, for example, a liquid, an emulsion, or a solid.

As used herein, the term "fermentation" refers to a process in which microorganisms break down organic materials using their own enzymes, excluding putrefaction. Fermentation and putrefaction proceed by similar processes, but when useful substances are produced as a result of decomposition, it is called fermentation, and when bad smells or harmful substances are produced, it is called putrefaction.

In the present disclosure, the method of obtaining the fermentation product from the microorganism is not particularly limited, and the fermentation product may be obtained according to a method commonly used in the art or similar art.

As used herein, the term "fermentation product" comprises not only a fermented material itself, but also all types of materials comprising the fermentation product generated from the microorganism, such as a substance comprising the fermented microorganism, a culture generated from the fermented microorganism, a fermentation product of the culture, a concentrated fermentation product, a dry product of the fermentation product, a filtrate of the fermentation product, a filtrate of the concentrated fermentation product, or a dry product of the filtrate of the fermentation product, an extract of the fermentation product, or a dilution of the fermentation product, etc.

In the method of the present disclosure, any culture conditions and culture methods known in the art may be used for culturing the microorganism. This culture process may be easily adjusted and used by those skilled in the art according to the selected microorganism.

The rebaudioside D and/or M produced by culturing of the present disclosure may be released into the medium or may remain in cells.

Still another aspect of the present disclosure provides a method of producing a steviol glycoside, the method comprising the step of transferring glucose to rebaudioside A by reacting rebaudioside A with nucleotide diphosphate-linked glucose in the presence of the variant polypeptide of the present disclosure and/or the microorganism of the present disclosure.

The steviol glycoside may be any one or more of rebaudioside D and rebaudioside M.

The variant polypeptide may be comprised in the microorganism of the present disclosure or may be obtained (e.g., purified) therefrom; and the reaction step may comprise a step of culturing the microorganism comprising any one or more of the variant polypeptide, the polynucleotide encoding the variant polypeptide, and the vector comprising the polynucleotide in a medium comprising nucleotide diphosphate-linked glucose and rebaudioside A.

The variant polypeptide, polynucleotide, vector, microorganism, culture, and medium, etc. are as explained in other aspects.

In one embodiment, the method of producing steviol glycoside may further comprise, but is not limited to, a step of producing nucleotide diphosphate-linked glucose by reacting sucrose with nucleotide diphosphate in the presence of sucrose synthase.

As used herein, the term "sucrose synthase" may play a role in producing sucrose by reversibly transferring nucleotide diphosphate-bound glucose to fructose in plant metabolisms, and in the present disclosure, it may exhibit an activity of separating nucleotide diphosphate-linked glucose and fructose by reacting sucrose with nucleotide diphosphate (e.g., in a pH range of 5 to 10).

The sucrose may be used without limitation as long as it may act as a substrate for sucrose synthase to provide glucose to nucleotide diphosphate. For example, raw sugar or sugar may be used, but is not limited thereto.

In the present disclosure, the nucleotide diphosphate may be a purine nucleotide or a pyrimidine nucleotide. Specifically, uridine diphosphate (UDP) may be used, but is not limited thereto.

In one embodiment, the method of producing steviol glycoside may further comprise a step of producing rebaudioside A by reacting nucleotide diphosphate-linked glucose with stevioside in the presence of glycosyltransferase A (UGT-A); and/or a microorganism comprising any one or more of glycosyltransferase A, a polynucleotide encoding the same, and a vector comprising the same.

In one embodiment of the aforementioned embodiments, the step of producing rebaudioside A may be, but is not limited to, a step of culturing the microorganism comprising any one or more of glycosyltransferase A (UGT-A) polypeptide, the polynucleotide encoding the same, and the vector comprising the same.

In one embodiment of the aforementioned embodiments, the glycosyltransferase A (UGT-A) may be glycosyltransferase derived from *Oryza sativa, Stevia rebaudiana Bertoni, Bambusa oldhamii, Brachypodium distachyon, Hordeum vulgare, Sorghum bicolor,* Zea *mays, Arabidopsis thaliana,* but is not limited thereto. The glycosyltransferase A (UGT-A) is not limited to its source as long as it has the activity of glycosyltransferase A (UGT-A), such as those produced from a recombinant microorganism (e.g., *Escherichia coli, Bacillus, yeast, Corynebacterium,* or *Agrobacterium)* transformed with the polynucleotide comprising the glycosyltransferase gene and/or the vector comprising the same, those obtained (e.g., purified) after production from the microorganism, those known, those commercially available, etc.

In one embodiment, the method of producing steviol glycoside and/or the step of producing rebaudioside A (or the method of producing rebaudioside A) may further comprise a step of producing nucleotide diphosphate-linked glucose by reacting sucrose with nucleotide diphosphate in the presence of sucrose synthase, but is not limited thereto.

In one embodiment, the production of rebaudioside A, D, and/or M may be performed continuously or discontinuously in the same reaction system, and may be performed in one-pot, but is not limited thereto.

In one embodiment, the method of producing steviol glycoside may further comprise a step of producing rebaudioside M by reacting rebaudioside D with nucleotide diphosphate-linked glucose in the presence of glycosyltransferase A (UGT-A); and/or the microorganism comprising any one or more of the glycosyltransferase A, the polynucleotide encoding the same, and the vector comprising the same.

In one specific embodiment, the method of producing steviol glycoside of the present disclosure may further comprise the steps of preparing the microorganism of the present disclosure, preparing a medium for culturing the microorganism, or a combination thereof (in any order), for example, before the culturing step.

The production method of the present disclosure may further comprise the step of recovering any one or more of rebaudioside D and rebaudioside M from the cultured microorganism, a culture of the microorganism, a fermentation product of the microorganism, the culture medium, or a reaction solution in which the enzymatic reaction has occurred. The recovering step may be further comprised after the culturing step.

The recovering may be collecting any one or more of desired rebaudioside D and rebaudioside M by using an appropriate method known in the art according to the method of culturing the microorganism of the present disclosure, for example, a batch, continuous, or fed-batch type culture. For example, centrifugation, filtration, treatment with a crystallized protein precipitating agent (salting-out), extraction, sonication, ultrafiltration, dialysis, various types of chromatography, such as molecular sieve chromatography (gel filtration), adsorption chromatography, ion exchange chromatography, and affinity chromatography, HPLC, and a combination of these methods may be used, and rebaudioside D and/or M may be recovered from the medium, the microorganism, or the reaction solution by using an appropriate method known in the art.

In addition, the method of producing steviol glycoside of the present disclosure may further comprise a purification step. The purification may be performed by using an appropriate method known in the art. In an exemplary embodiment, when the method of producing steviol glycoside of the present disclosure comprises both the recovering step and the purification step, the recovering step and the purification step may be performed continuously or discontinuously regardless of the order, or may be performed simultaneously or integrated into one step, but is not limited thereto.

Still another aspect of the present disclosure provides a method of producing rebaudioside D, the method comprising the step of producing rebaudioside D by reacting nucleotide diphosphate-linked glucose with rebaudioside A in the presence of the variant polypeptide of the present disclosure and/or the microorganism of the present disclosure.

The method of producing rebaudioside D may comprise the method (step) of producing rebaudioside A provided in the above-described aspect, and the glycosyltransferase A (UGT-A) is as explained in other aspects.

In one embodiment, the method of producing rebaudioside D may further comprise the step of recovering rebaudioside D.

In one embodiment, the production of rebaudioside A and/or D may be performed continuously or discontinuously in the same reaction system, but is not limited thereto.

Still another aspect of the present disclosure provides a method of producing rebaudioside M, the method comprising the step of producing rebaudioside M by reacting rebaudioside D with nucleotide diphosphate-linked glucose in the presence of glycosyltransferase A (UGT-A); and/or a microorganism comprising any one or more of glycosyltransferase A, a polynucleotide encoding the same, and a vector comprising the same.

The method of producing rebaudioside M may comprise the method (step) of producing rebaudioside A and/or the method of producing rebaudioside D (the step of producing rebaudioside D) provided in the above-described aspect.

The method of producing rebaudioside A, the method of producing rebaudioside D, and the glycosyltransferase A (UGT-A) are as explained in other aspects.

In one embodiment, the method of producing rebaudioside M may further comprise the step of recovering rebaudioside M.

In one embodiment, the production of rebaudioside A, D, and/or M may be performed continuously or discontinuously in the same reaction system, but is not limited thereto.

Still another aspect of the present disclosure provides a composition for producing any one or more of rebaudioside D and rebaudioside M, the composition comprising the variant polypeptide of the present disclosure; the polynucleotide encoding the variant polypeptide; the microorganism comprising any one or more of the variant polypeptide, the polynucleotide encoding the variant polypeptide, and the vector comprising the polynucleotide; the culture of the microorganism; or any one or more thereof.

The variant polypeptide, polynucleotide, vector, microorganism, and culture, etc. are as explained in other aspects.

The composition of the present disclosure may further comprise any appropriate excipient that is commonly used in the composition for producing rebaudioside D and/or M, and examples of the excipient may comprise a preserving agent, a wetting agent, a dispersing agent, a suspending agent, a buffering, a stabilizing agent, an isotonic agent, etc., but are not limited thereto.

In one specific embodiment, each component present in the composition of the present disclosure may be comprised in a microbiologically effective amount, or in an amount that may be appropriately present in the composition for production.

Still another aspect of the present disclosure provides use of the variant polypeptide of the present disclosure or the microorganism of the present disclosure in producing rebaudioside D and/or M. Specifically, the aspect provides use of the microorganism comprising any one or more of the variant polypeptide of the present disclosure, the polynucleotide encoding the variant polypeptide, and the vector comprising the polynucleotide in producing any one or more of rebaudioside D and rebaudioside M.

The variant polypeptide and microorganism, etc. are as explained in other aspects.

Still another aspect of the present disclosure provides use of the composition comprising the variant polypeptide of the present disclosure; the polynucleotide encoding the variant polypeptide; the microorganism comprising any one or more of the variant polypeptide, the polynucleotide encoding the variant polypeptide, and the vector comprising the polynucleotide; the culture of the microorganism; or any one or more thereof in producing any one or more of rebaudioside D and rebaudioside M.

The variant polypeptide, polynucleotide, microorganism, and culture, etc. are as explained in other aspects.

### [Mode for Carrying Out the Invention]

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

### Example 1. Design and Preparation of UGT-B improved enzymes

### 1-1. Design of UGT-B improved enzymes

To increase the activity of UGT-B enzyme, a total of 26 variants were designed, based on the amino acid sequence of SEQ ID NO: 1, as shown in Table 1 below.

**[Table 1]**

| **Name** | **Substitution** |
|---|---|
| M1 | Y92W |
| M2 | Y208A |
| M3 | Y208M |
| M4 | F380H |
| M5 | F380T |
| M6 | F380A |
| M7 | G381F |
| M8 | G381S |
| M9 | G381A |
| M10 | G381N |
| M11 | F380H-G381F |
| M12 | F380H-G381N |
| M13 | F380H-G381S |
| M14 | F380H-G381A |
| M15 | F380G-G381F |
| M16 | F380G-G381N |
| M17 | F380G-G381S |
| M18 | F380G-G381A |
| M19 | F380T-G381F |
| M20 | F380T-G381N |
| M21 | F380T-G381S |
| M22 | F380T-G381A |
| M23 | F380A-G381F |
| M24 | F380A-G381N |
| M25 | F380A-G381S |
| M26 | F380A-G381A |

### 1-2. Preparation of UGT-B improved enzymes

The improved enzymes designed in Example 1-1 were prepared by a site-directed mutagenesis method. In the site-directed mutagenesis PCR reaction, AccuPower^{®} Pfu PCR premix (Bioneer), 10 ng of a template plasmid (*E. coli-Corynebacterium* shuttle vector pCES208; or pET-28a vector for expression in *E. coli*), and each 0.5 µM of forward and reverse primers were used (primers as shown in Table 2 below were used depending on the type of improved enzyme). In addition, the above-mentioned PCR was performed using a Mastercycler^{®} X50s (Eppendorf) under the following conditions: pre-denaturation at 94°C for 3 minutes; 20 cycles of denaturation at 94°C for 30 seconds, annealing at 58°C for 30 seconds, and extension at 72°C for 1 minute/kb; and final extension at 72°C for 5 minutes.

Thereafter, 1 µl of DpnI (NEB, R0176L) was used to digest the template plasmid, and 5 µl of the PCR reaction solution was injected into *E. coli* DH5α strain using a heat-shock method.

Each of the transformed *E. coli* strains was cultured in LB medium comprising 50 mg/L kanamycin. After colony selection, mutations were identified through plasmid DNA sequencing (Bioneer).

Two types of plasmid vectors were used in the preparation of the improved enzymes. One is the *E. coli*-*Corynebacterium* shuttle vector (pCES208), which comprises a UGT-B gene sequence (SEQ ID NO: 2) codon-optimized for expression in *Corynebacterium* strains, and is referred to as pCES_UGT-B. The other vector is the pET-28a vector for expression in *E. coli*, which comprises a UGT-B gene sequence (SEQ ID NO: 127) codon-optimized for expression in *E. coli* strains with a 6x his tag for purification. Both vectors comprise a kanamycin resistance gene.

**[Table 2]**

| **Name** | **Forward primer sequence** | **Reverse primer sequence** | **SEQ ID NO.** |
|---|---|---|---|
| M1 | GATGTCCCATGGGATAAATTC GAGTTAC | GAATTTATCCCATGGGACATCG TTGGTTG | 75, 76 |
| M2 | TAGCCGAACGCGCCTTCTTAA CTTTAATGC | AAGGCGCGTTCGGCTACCGTC ATC | 77.78 |
| M3 | TAGCCGAACGCATGTTCTTAA CTTTAATGC | GAACATGCGTTCGGCTACCGT CATC | 79,80 |
| M4 | TATCCACGGTGATCAGGGCC CAAAC | TGATCACCGTGGATAGGGAGC ATCAC | 81,82 |
| M5 | CTCCCTATCACCGGTGATCA G | TCACCGGTGATAGGGAGCATC | 83,84 |
| M6 | ATGCTCCCTATCGCTGGTGAT CAG | ACCAGCGATAGGGAGCATCAC TAAG | 85,86 |
| M7 | ATCTTCTTTGATCAGGGCCCA AAC | GATCAAAGAAGATAGGGAGCA TCAC | 87,88 |
| M8 | ATCTTCTCTGATCAGGGCCCA AAC | GATCAGAGAAGATAGGGAGCA TCAC | 89,90 |
| M9 | TTCGCTGATCAGGGCCCAAA C | GATCAGCGAAGATAGGGAGCA TC | 91,92 |
| M10 | ATCTTCAATGATCAGGGCCCA AAC | GATCATTGAAGATAGGGAGCA TCAC | 93,94 |
| M11 | TATCCACTTTGATCAGGGCCC AAAC | TGATCAAAGTGGATAGGGAGC ATCAC | 95,96 |
| M12 | TATCCACAATGATCAGGGCC CAAAC | TGATCATTGTGGATAGGGAGC ATCAC | 97,98 |
| M13 | TATCCACTCTGATCAGGGCC CAAAC | TGATCAGAGTGGATAGGGAGC ATCAC | 99,100 |
| M14 | TATCCACGCTGATCAGGGCC CAAAC | TGATCAGCGTGGATAGGGAGC ATCAC | 101,102 |
| M15 | TATCGGCTTTGATCAGGGCC CAAAC | TGATCAAAGCCGATAGGGAGC ATCAC | 103,104 |
| M16 | TATCGGTAATGATCAGGGCC CAAAC | TGATCATTACCGATAGGGAGC ATCAC | 105,106 |
| M17 | TATCGGTTCTGATCAGGGCC CAAAC | TGATCAGAACCGATAGGGAGC ATCAC | 107,108 |
| M18 | TATCGGTGCTGATCAGGGCC CAAAC | TGATCAGCACCGATAGGGAGC ATCAC | 109,110 |
| M19 | TATCACCTTTGATCAGGGCCC AAAC | ATCAAAGGTGATAGGGAGCAT CAC | 111,112 |
| M20 | TATCACCAATGATCAGGGCC CAAAC | GATCATTGGTGATAGGGAGCA TCAC | 113,114 |
| M21 | TATCACCTCTGATCAGGGCC CAAAC | TGATCAGAGGTGATAGGGAGC ATCAC | 115,116 |
| M22 | TATCACCGCTGATCAGGGCC CAAAC | TGATCAGCGGTGATAGGGAGC ATC | 117,118 |
| M23 | TATCGCTTTTGATCAGGGCCC AAAC | TGATCAAAAGCGATAGGGAGC ATCAC | 119,120 |
| M24 | TATCGCTAATGATCAGGGCC CAAAC | TGATCATTAGCGATAGGGAGC ATCAC | 121,122 |
| M25 | TATCGCTTCTGATCAGGGCC CAAAC | TGATCAGAAGCGATAGGGAGC ATCAC | 123,124 |
| M26 | TATCGCTGCTGATCAGGGCC CAAAC | TGATCAGCAGCGATAGGGAGC ATCAC | 125,126 |

### Example 2. Preparation of Microorganisms Expressing Improved Enzymes and Comparison of RebD Synthesis Ability between 26 Types of Improved Enzymes

26 types of the improved enzymes prepared in Example 1 were expressed in *Corynebacterium ammoniagenes* (hereinafter, referred to as *C.amm),* and the cell reaction was performed to compare the conversion rate of rebaudioside A (hereinafter, referred to as RebA) into rebaudioside D (hereinafter, referred to as RebD).

To this end, the expression vector for *Corynebacterium* microorganisms, which is the plasmid vector of the improved enzymes prepared in Example 1, was first inserted into the wild-type *C.amm* strain ATCC6872 using electroporation.

The *C.amm* strain transformed with the plasmid vector was cultured on a solid medium (80 g/L of glucose, 20 g/L of soytone, 10 g/L of (NH₄)₂SO₄, 1.2 g/L of KH₂PO₄, 1.4 g/L of MgSO₄, 15 g/L of agar) comprising 50 µg/ml of kanamycin. For the expression of the improved enzymes, colonies grown on the solid medium were inoculated into 400 µl of a medium for *Corynebacterium* microorganism (80 g/L of glucose, 20 g/L of soytone, 10 g/L of (NH₄)₂SO₄, 1.2 g/L of KH₂PO₄, 1.4 g/L of MgSO₄) comprising 50 µg/ml kanamycin, and pre-cultured in a 96-well plate. This pre-culture was performed using a shaking incubator (Infors HT Multitron Incubator Shaker) at 30°C and 800 rpm for 24 hours, and then 10% (v/v) of the total volume of the pre-culture was inoculated into a fresh medium for *Corynebacterium* (comprising 50 µg/ml of kanamycin) (80 g/L of glucose, 20 g/L of soytone, 10 g/L of (NH₄)₂SO₄, 1.2 g/L of KH₂PO₄, 1.4 g/L of MgSO₄) and cultured under the same conditions for 24 hours.

At this time, to confirm reproducibility, two colonies per improved enzyme were cultured for the strains comprising each of the improved enzymes, and four colonies were cultured for the strain comprising the wild-type UGT-B for comparison.

For an enzyme activity test based on the RebD synthesis ability, 200 µl of the culture of each strain comprising the improved enzyme or the wild-type UGT-B was placed in a 96-well plate, and centrifuged to discard the supernatant. A total of 400 µl of a reaction solution comprising 10 mM uridine-5'-diphosphate-glucose (UDPG; Carbosynth), 10 % (w/v) RebA (Daepyung), 0.8 % POESA (v/v), and 50 mM potassium phosphate (pH 7.0) was added to the remaining cells, and allowed to react at 40°C for 24 hours. The reaction solution was then diluted 1:10 with triple-distilled water, and the enzyme was inactivated at 100°C for 5 minutes, and pretreated using a filter. The converted RebD was analyzed from the pretreated reaction solution using HPLC (Agilent). The HPLC analysis conditions were as follows.
- Detector wavelength: 210 nm
- Flow rate: 1 ml/min
- Sample injection volume: 10 µl
- Column: Capcell pak C18 MG II (Shiseido, 250 x 4.6 mm, particle size: 5 µm)
- Solvent: Acetonitrile 30%

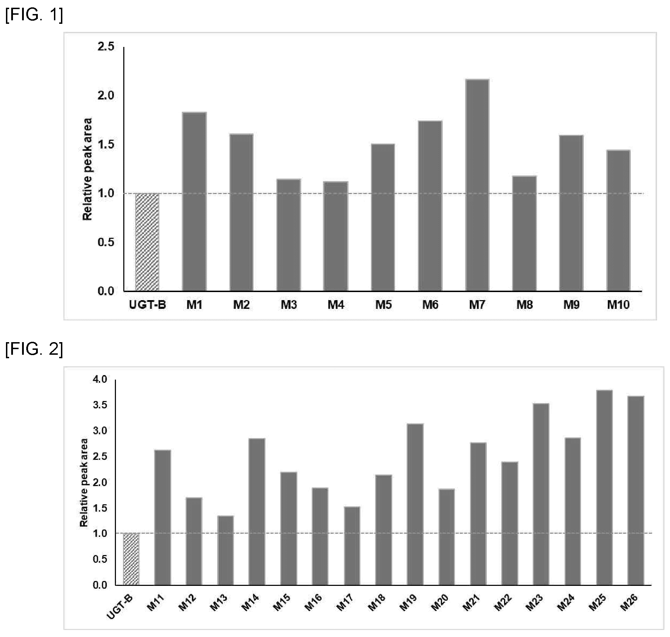

The analysis results are shown in FIGS. 1 and 2.

As a result, as shown in FIG. 1, all the prepared single variants (M1, M2, M3, M4, M5, M6, M7, M8, M9 and M10) were confirmed to have superior RebD conversion activity, compared to the wild-type UGT-B.

In addition, as shown in FIG. 2, all the double variants (M11 - M26) of F380X - G381X, prepared based on the results of the activities of the single variants, were confirmed to have superior activities, compared to the wild-type UGT-B and each single variant on which the double variant was based.

### Example 3. Comparison of Activities between UGT-B (WT) and Improved Enzymes

### 3-1. Assessment of RebD Synthesis Activity of UGT-B Improved Enzymes

Based on the results of Example 2, 7 types (M1, M2, M14, M19, M20, M21, M26) were selected from the improved enzymes with superior activity compared to wild-type UGT-B and their activity for RebA was assessed.

To this end, the recombinant *Corynebacterium* microorganism prepared in Example 2 was inoculated into a medium comprising 50 µg/ml of kanamycin at an initial concentration of O.D. 600=0.1, and pre-cultured at 30°C for 24 hours. 5 ml of the obtained pre-culture solution was then inoculated into 50 ml of a medium comprising 50 µg/ml of kanamycin, and cultured at 30°C for 24 hours to induce enzyme expression. Thereafter, the cells were separated from the culture using centrifugation.

The enzymatic reaction was performed at 30°C for 16 hours by mixing 100 mg/ml of *Corynebacterium* microorganism cells in a reaction solution comprising 10 mM UDPG (Carbosynth), RebA (Daepyung), 0.8 % POESA (v/v), and 50 mM potassium phosphate (pH 7.0). After completion of the enzymatic reaction, the HPLC analysis method was performed in the same manner as in Example 2, and as a result, the conversion rate into RebD relative to that of the wild-type UGT-B is shown in Table 3 below.

**[Table 3]**

| Raw material | UGT-B | M1 | M2 | M14 | M19 | M20 | M21 | M26 |
|---|---|---|---|---|---|---|---|---|
| 1 % (w/v) RebA | 100 | 150 | 151 | 170 | 218 | 195 | 209 | 210 |
| 3 % (w/v) RebA | 100 | 187 | 161 | 178 | 248 | 203 | 244 | 244 |

As shown in Table 3, it was confirmed that all the tested improved enzymes have remarkably superior conversion rates into RebD, compared to the wild-type UGT-B.

### 3-2. Assessment of RebDM Synthesis Activity of UGT-B Improved Enzymes (1)

When glycosyltransferase UGT-A, sucrose synthase (SS), and glycosyltransferase UGT-B are used, raw materials such as stevioside may be converted into RebD and rebaudioside M (hereinafter, referred to as RebM) using sucrose and uridine-5'-diphosphate (UDP) instead of uridine-5'-diphosphate-glucose (UDPG).

Therefore, in this Example, a reaction of converting a raw material SG90 (Haigen), in which stevioside and RebA are mixed, into RebD and RebM was assessed using a mixture of recombinant *Corynebacterium* microorganism expressing UGT-A and SS and recombinant *Corynebacterium* microorganism expressing UGT-B improved enzyme. To this end, a gene (SEQ ID NO: 130) encoding UGT-A (SEQ ID NO: 129) and a gene (SEQ ID NO: 132) encoding SS (SEQ ID NO: 131) were cloned into the *E. coli*-*Corynebacterium* shuttle vector (pCES208). This was transformed into the *Corynebacterium* microorganism in the same manner as described in Example 2 to produce a strain simultaneously expressing UGT-A and SS. The vector which was prepared and used for the transformation is called pCES_SS_UGT-A. A method of culturing the *Corynebacterium* microorganism expressing the UGT-B improved enzyme (representatively M20, M21, M26) for enzyme expression was performed in the same manner as described in Example 3-1.

The enzymatic reaction was performed at 30°C for 24 hours by mixing each 100 mg/ml of UGT-A and SS expressing cells and UGT-B expressing cells in a reaction solution comprising 10 mM UDP (Carbosynth), 1% (w/v) SG90 (Haigen), 10% (w/v) 0.8% POESA (v/v), and 50 mM potassium phosphate (pH 8.0). After completion of the enzymatic reaction, the HPLC analysis method was performed in the same manner as in Example 2, and as a result, the conversion rate into RebD and RebM mixture (RebDM) relative to that of the wild-type UGT-B is shown in Table 4 below.

**[Table 4]**

| Raw material | UGT-B (WT) | M20 | M21 | M26 |
|---|---|---|---|---|
| 1% SG90 | 100 | 175 | 192 | 185 |

As shown in Table 4, it was confirmed that the improved enzymes have remarkably superior conversion rates into RebD and RebM mixture, compared to the wild-type UGT-B.

### Example 4. Comparison of Activities between UGT-B (WT) and Complex Improved Enzymes

### 4-1. Preparation of UGT-B Complex Improved Enzymes

In order to analyze the synergistic effect between mutations of the improved enzymes, of which increased activities were confirmed when compared to that of UGT-B, combinatorial mutants comprising two or more mutations were designed as shown in Table 5 below.

**[Table 5]**

| Name | Substitution |
|---|---|
| MM1 | Y208A-F380A-G381A |
| MM2 | Y208A-F380T-G381F |
| MM3 | Y208A-F380T-G381S |
| MM4 | Y92W-F380A-G381A |
| MM5 | Y92W-F380T-G381F |
| MM6 | Y92W-F380T-G381S |
| MM7 | Y92W-Y208A |
| MM8 | Y92W-Y208A-F380A-G381A |
| MM9 | Y92W-Y208A-F380T-G381F |
| MM10 | Y92W-Y208A-F380T-G381S |

The complex improved enzymes designed as above were prepared by the site-directed mutagenesis PCR method as in Example 1-2.

In detail, each of the plasmids comprising the sequences of the improved enzyme variants prepared in Example 1-2 was used as a template and one pair or two pairs of primers for additional mutations were used to prepare additional remaining mutations by the site-directed mutagenesis PCR method. For example, in the case of the MM1 variant, a plasmid comprising a polynucleotide sequence of the Y208A (M2) variant was used as a template and primers for M26 were used to additionally prepare F380A-G381A. For another example, in the case of the MM10 variant, a plasmid comprising a polynucleotide sequence of the Y92W (M1) variant was used as a template and primers for M2 and primers for M31 were used to comprise Y208A and additional F380T-G381S mutations.

### 4-2. Assessment of RebD Synthesis Activity of Complex Improved Enzymes

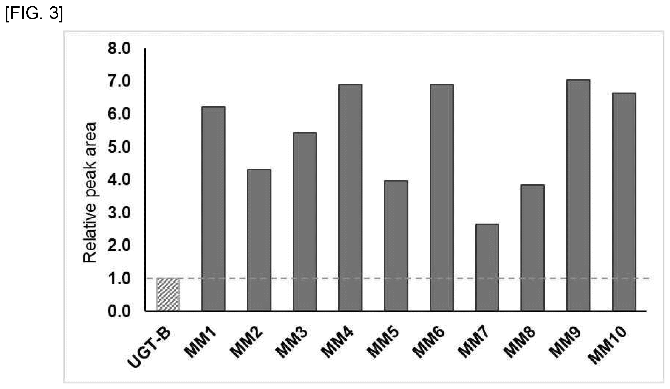

Recombinant *Corynebacterium* strains expressing each of the complex improved enzymes prepared as in Example 4-1 were prepared by the same transformation method as in Example 2, and the activity assessment using the same was performed in the same manner as in Example 3-1, and the results are shown in FIG. 3.

As a result, as shown in FIG. 3, it was confirmed that all the prepared complex improved enzymes have remarkably superior enzyme activities, compared to the wild-type UGT-B.

### Example 5. Assessment of RebDM Synthesis Activity of Improved Enzymes (2)

Additionally, the RebDM synthesis activities of the improved enzymes were assessed in a different way. Four types of *E. coli* BL21(DE3) strains were each prepared, into which for the expression of four types of enzymes (UGT-A, UGT-B_WT, UGT-B_M21, and RSS), each vector prepared by cloning pET28-α with the gene (SEQ ID NOS: 130, 127, 128, or 132) encoding the enzyme was introduced. Each strain was inoculated into 5 ml of LB medium and cultured at 37°C and 200 rpm for 8 hours, and then added to 2 L of LB medium to perform a main culture. After culturing at 37°C and 200 rpm until absorbance of the culture reached 0.4 at 600 mm, 1 mM isopropyl β-D-1-thiogalactothiopyranoside (IPTG) was added to induce mass-expression of the enzyme. After adding IPTG, the culture was performed at 20°C and 120 rpm for about 16 hours. The culture was centrifuged at 6000 x g for 20 minutes at 4°C to obtain the cells, and the soluble proteins each for the four enzymes mentioned above were extracted through ultrasonication, and each enzyme was purified using Ni-NTA affinity chromatography.

The purified enzyme and the raw material (SG90, Haigen) comprising stevioside and RebA as the main components were used to perform the enzymatic reaction at 40°C and pH 8.0 to 9.0 under conditions 1 to 3 as shown in Table 6 below.

**[Table 6]**

| Composition | Condition 1 | Condition 2 | Condition 3 |
|---|---|---|---|
| SG90 raw material concentration | 3% | 5% | 10% |
| UDP concentration | 10 Mm | | |
| Sucrose concentration | 30% | | |
| Na₂HPO₄ (pH8.0) | 100 mM | | |
| SS concentration (mg/ml) | 0.2 | 0.3 | 0.4 |
| UGT-A concentration (mg/ml) | 1.0 | 2.0 | 4.0 |
| UGT-B_WT or M21 concentration (mg/ml) | 0.2 | 0.4 | 0.8 |

Then, after enzyme inactivation at 100°C for 20 minutes, the production amounts of RebD, RebM, and RebDM (a mixture of RebD and RebM) when UGT-B_WT and UGT-B_M21 were added were compared through HPLC analysis in the same manner as Example 2, respectively.

In the case of the reaction solution to which UGT-B_M21 enzyme was added under conditions 1 to 3, the RebDM amount was 100%, 100%, and 90.8%, respectively, and the RebM amount was 89.5%, 83.8%, and 52.1%, respectively, after 72 hours of reaction, and the results are shown in Table 7 below.

**[Table 7]**

| Raw material% (w/v) | UGT-B | 24-hr reaction | | | 48-hr reaction | | | 72-hr reaction | | |
|---|---|---|---|---|---|---|---|---|---|---|
| | | RebD | RebM | RebDM | RebD | RebM | RebDM | RebD | RebM | RebDM |
| 3% | WT | 5.2% | 18.3% | 23.5% | 5.4% | 24.3% | 29.7% | 5.9% | 32.2% | 38.1% |
| | **M21** | **28.5 %** | **43.9%** | **72.5%** | **25.8 %** | **68.3%** | **94.1%** | **10.5%** | **89.5%** | **100.0%** |
| 5% | WT | 6.6% | 19.3% | 25.9% | 8.4% | 22.8% | 31.3% | 8.4% | 36.9% | 45.3% |
| | **M21** | **36.7 %** | **43.0%** | **79.7%** | **32.5 %** | **64.4%** | **96.9%** | **16.2%** | **83.8%** | **100.0%** |
| 10% | WT | 7.2% | 16.7% | 23.9% | 9.7% | 16.6% | 26.3% | 8.9% | 24.0% | 32.9% |
| | **M21** | **36.6 %** | **27.2%** | **63.8%** | **43.3 %** | **37.4%** | **80.7%** | **38.7%** | **52.1%** | **90.8%** |

When the results of the reaction solution, to which the UGT-B_M21 enzyme was added, were compared with the results of the reaction solution to which the UGT-B_WT enzyme was added, both the RebDM amount and the RebM amount were about 2.2 times to 2.8 times higher, demonstrating that the enzyme's ability to convert RebA into RebD was remarkably improved, and it is advantageous to use the improved enzyme of the present disclosure in RebM conversion through a complex reaction.

From the above results, it was confirmed that RebDM production may be increased using the UGT-B single and complex variant enzymes of the present disclosure.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A variant polypeptide having glycosyltransferase activity, the variant polypeptide comprising any one or more of the following: a substitution of an amino acid corresponding to position 92 of an amino acid sequence of SEQ ID NO: 1 with tryptophan; a substitution of an amino acid corresponding to position 208with alanine or methionine; a substitution of an amino acid corresponding to position 380 with histidine, threonine, alanine, or glycine; and a substitution of an amino acid corresponding to position 381 with phenylalanine, serine, alanine, or asparagine.

2. The variant polypeptide of claim 1, wherein the variant polypeptide comprises
a substitution of the amino acid corresponding to position 92 of the amino acid sequence of SEQ ID NO: 1 with tryptophan;
a substitution of the amino acid corresponding to position 208 with alanine or methionine;
a substitution of the amino acid corresponding to position 380 with histidine, threonine, or alanine;
a substitution of the amino acid corresponding to position 381 with phenylalanine, serine, alanine, or asparagine; or
a substitution of the amino acid corresponding to position 380 with histidine, threonine, or glycine and a substitution of the amino acid corresponding to position 381 with phenylalanine, serine, alanine, or asparagine.

3. The variant polypeptide of claim 1, wherein the variant polypeptide comprises
a substitution of the amino acid corresponding to position 208 with alanine, a substitution of the amino acid corresponding to position 380 with threonine or alanine, and a substitution of the amino acid corresponding to position 381 with phenylalanine, serine, or alanine;
a substitution of the amino acid corresponding to position 92 with tryptophan, a substitution of the amino acid corresponding to position 380 with threonine or alanine, and a substitution of the amino acid corresponding to position 381 with phenylalanine, serine, or alanine;
a substitution of the amino acid corresponding to position 92 with tryptophan, and a substitution of the amino acid corresponding to position 208 with alanine; or
a substitution of the amino acid corresponding to position 92 with tryptophan, a substitution of the amino acid corresponding to position 208 with alanine, a substitution of the amino acid corresponding to position 380 with threonine or alanine, and a substitution of the amino acid corresponding to position 381 with phenylalanine, serine, or alanine.

4. The variant polypeptide of claim 1, wherein the variant polypeptide has 80% or more homology or identity with SEQ ID NO: 1.

5. A polynucleotide encoding the variant polypeptide of any one of claims 1 to 4.

6. A vector comprising the polynucleotide of claim 5.

7. A microorganism comprising any one or more of the variant polypeptide of any one of claims 1 to 4, a polynucleotide encoding the variant polypeptide, and a vector comprising the polynucleotide.

8. The microorganism of claim 7, wherein the microorganism has an enhanced ability to produce any one or more of rebaudioside D and rebaudioside M, compared to a microorganism comprising a polypeptide of an amino acid sequence of SEQ ID NO: 1 or a polynucleotide encoding the same.

9. The microorganism of claim 7, wherein the microorganism is a microorganism of *Corynebacterium* sp. or *Escherichia* sp.

10. The microorganism of claim 9, wherein the microorganism is *Corynebacterium ammoniagenes, Corynebacterium glutamicum,* or *Escherichia coli.*

11. A method of producing a steviol glycoside, the method comprising the step of transferring glucose to rebaudioside A by reacting rebaudioside A with a nucleotide diphosphate-linked glucose in the presence of the variant polypeptide of claim 1 or the microorganism of claim 7, wherein the steviol glycoside is any one or more of rebaudioside D and rebaudioside M.

12. The method of claim 11, further comprising the step of recovering any one or more of rebaudioside D and rebaudioside M.

13. The method of claim 11, further comprising the step of producing rebaudioside M by reacting rebaudioside D with nucleotide diphosphate-linked glucose in the presence of glycosyltransferase A (UGT-A); or a microorganism comprising any one or more of the glycosyltransferase A, a polynucleotide encoding the same, and a vector comprising the same.

14. A composition for producing any one or more of rebaudioside D and rebaudioside M, the composition comprising any one or more of the variant polypeptide of any one of claims 1 to 4; a polynucleotide encoding the variant polypeptide; a microorganism comprising any one or more of the variant polypeptide, the polynucleotide encoding the variant polypeptide, and a vector comprising the polynucleotide; a culture of the microorganism; or any one or more thereof

15. Use of a microorganism comprising any one or more of the variant polypeptide of any one of claims 1 to 4, a polynucleotide encoding the variant polypeptide, and a vector comprising the polynucleotide in producing any one or more of rebaudioside D and rebaudioside M.

16. Use of a composition comprising the variant polypeptide of any one of claims 1 to 4; a polynucleotide encoding the variant polypeptide; a microorganism comprising any one or more of the variant polypeptide, the polynucleotide encoding the variant polypeptide, and a vector comprising the polynucleotide; a culture of the microorganismor any one or more thereof in producing any one or more of rebaudioside D and rebaudioside M.
